(19) Europäisches Patentamt
European Patent Office
Office européen des brevets

(11) **EP 3 868 779 B1**

(12) **EUROPEAN PATENT SPECIFICATION**

(45) Date of publication and mention
of the grant of the patent:
**06.11.2024 Bulletin 2024/45**

(21) Application number: **19874354.4**

(22) Date of filing: **15.10.2019**

(51) International Patent Classification (IPC):
**A61L 27/22** (2006.01)    **A61L 27/52** (2006.01)
**A61L 27/54** (2006.01)    **B33Y 70/00** (2020.01)
**C07K 14/435** (2006.01)    **C09D 11/08** (2006.01)

(52) Cooperative Patent Classification (CPC):
**A61L 27/52; A61L 27/227; A61L 27/54;**
**B33Y 70/00; C07K 14/43586; C09D 11/08;**
A61L 2300/214; A61L 2300/252; A61L 2300/64;
C07K 2319/00; C07K 2319/73

(86) International application number:
**PCT/ES2019/070701**

(87) International publication number:
**WO 2020/079303 (23.04.2020 Gazette 2020/17)**

(54) **COMPOSITION BASED ON RECOMBINANT BIOPOLYMERS AND USES OF SAME AS BIO-INK**

ZUSAMMENSETZUNG AUF BASIS VON REKOMBINANTEN BIOPOLYMEREN UND
VERWENDUNGEN DAVON ALS BIOTINTE

COMPOSITION À BASE DE BIOPOLYMÈRES RECOMBINÉS ET UTILISATIONS DE CELLE-CI
COMME ENCRE BIOLOGIQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **17.10.2018 ES 201831008**

(43) Date of publication of application:
**25.08.2021 Bulletin 2021/34**

(73) Proprietor: **Universidad de Valladolid**
**47002 Valladolid (ES)**

(72) Inventors:
• **SALINAS FERNÁNDEZ, Soraya**
**47002 Valladolid (ES)**
• **RODRIGUEZ CABELLO, José Carlos**
**47002 Valladolid (ES)**
• **ALONSO RODRIGO, Matilde**
**47002 Valladolid (ES)**
• **SANTOS GARCIA, Mercedes**
**47002 Valladolid (ES)**

(74) Representative: **Pons**
**Glorieta Rubén Darío 4**
**28010 Madrid (ES)**

(56) References cited:
EP-A1- 2 641 965    WO-A1-2014/041231
WO-A1-2014/102547    WO-A1-2016/019078
WO-A1-2017/095782    WO-A2-2015/168321
ES-B1- 2 344 097

• **FERNÁNDEZ-COLINO ALICIA ET AL:**
**"Self-Organized ECM-Mimetic Model Based on an**
**Amphiphilic Multiblock Silk-Elastin-Like**
**Corecombinamer with a Concomitant Dual**
**Physical Gelation Process",**
**BIOMACROMOLECULES, vol. 15, no. 10, 13**
**October 2014 (2014-10-13), US, pages 3781 - 3793,**
**XP055860457, ISSN: 1525-7797, DOI:**
**10.1021/bm501051t**
• **ALICIA FERNÁNDEZ-COLINO ET AL:**
**"Amphiphilic Elastin-Like Block**
**Co-Recombinamers Containing Leucine Zippers:**
**Cooperative Interplay between Both Domains**
**Results in Injectable and Stable Hydrogels",**
**BIOMACROMOLECULES, vol. 16, no. 10, 29**
**September 2015 (2015-09-29), US, pages 3389 -**
**3398, XP055704107, ISSN: 1525-7797, DOI:**
**10.1021/acs.biomac.5b01103**

- DINJASKI NINA ET AL: "Recombinant protein blends: silk beyond natural design", CURRENT OPINION IN BIOTECHNOLOGY, LONDON, GB, vol. 39, 10 December 2015 (2015-12-10), pages 1 - 7, XP029569332, ISSN: 0958-1669, DOI: 10.1016/J.COPBIO.2015.11.002
- CHOW D ET AL: "Peptide-based biopolymers in biomedicine and biotechnology", MATERIALS SCIENCE AND ENGINEERING: R: REPORTS, ELSEVIER, AMSTERDAM, NL, vol. 62, no. 4, 1 September 2008 (2008-09-01), pages 125 - 155, XP023976566, ISSN: 0927-796X, [retrieved on 20080803], DOI: 10.1016/J.MSER.2008.04.004
- FERNANDEZ-COLINO, ALICIA ET AL.: "Amphiphilic elastin-like block co-recombinamers containing leucine zippers: Cooperative interplay between both domains results in injectable and stable hydrogels", BIOMACROMOLECULES, vol. 16, no. 10, 2015, pages 3389 - 3398, XP055704107, DOI: 10.1021/acs.biomac.5b01103
- PEREZ DEL RIO, EDUARDO ET AL.: "Síntesis y caracterización de nuevos polímeros recombinantes tipo elastina fusionados a eGFP para la formation de nanopartículas", 2015, pages 3 , 5 - 17 y 25-28, XP055704109
- ZOU, YU ET AL.: "Diseño y síntesis de un nuevo biomaterial recombinante para uso en regeneration tisular neuronal", 2017, pages 1 - 2, 8-18, XP055704113
- IBÁÑEZ-FONSECA, A. ET AL.: "NOVEL HYDROGEL-FORMING ELASTIN-LIKE RECOMBINAMERS FOR BIOMEDICAL APPLICATIONS", CONGRESO ANUAL DE LA SOCIEDAD ESPANOLA DE INGENIERÍA BIOMÉDICA, November 2015 (2015-11-01), pages 331 - 333, XP055704115
- MISBAH, M. HAMED ET AL.: "Formation of calcium phosphate nanostructures under the influence of self-assembling hybrid elastin-like-statherin recombinamers", RSC ADVANCES, vol. 6, no. 37, 2016, pages 31225 - 31234, XP055704120, DOI: 10.1039/C6RA01100D
- JAVIER ARIAS, F. ET AL.: "Recent contributions of elastin-like recombinamers to biomedicine and nanotechnology", CURRENT TOPICS IN MEDICINAL CHEMISTRY, vol. 14, no. 6, 2014, pages 819 - 836, XP055704121

Remarks:

The complete document including Reference Table(s) and the Sequence Listing(s) can be downloaded from the EPO website

## Description

The present invention relates to compositions comprising recombinant biopolymers synthesized from elastin-like recombinamer (ELR) monomers, which comprise a sequence called "*silk*" originating from the silkworm *Bombyx mori,* and/or monomers comprising a sequence called HLF and which belongs to a natural class of proteins called *zippers.* These compositions are useful as bio-ink for 3D printing. In addition, the invention relates to methods for obtaining the composition of the invention, to the 3D biomaterial and to different uses of the composition and of the biomaterial comprising same.

## BACKGROUND OF THE INVENTION

3D bioprinting techniques include stereolithography, inkjet-based bioprinting, laser-based bioprinting and extrusion-based bioprinting, with the latter technique being the most widely used. In extrusion-based bioprinting, a hydrogel is introduced inside a cartridge and extruded by pressure onto a surface. Printed structures are made by layer-by-layer deposition of the material by spatial and temporal movement control by means of CAM - CAD (Computer Aid Manufacturing - Computer Aid Design) software.

Until now, considerable research has been performed using the extrusion technique to mimic tissues such as bone, heart tissue, cartilage, liver, lung, nervous tissue, skin and pancreatic tissue (Ozbolat, I.T., et al. Drug Discovery Today, 2016. 21(8): p. 1257-1271). It has also been used to perform *in vitro* modelling of diseases and drug release (Vanderburgh, J., et al. Ann Biomed Eng, 2017. 45(1): p. 164-179).

The parameters to be fulfilled by a material so that it can be used in 3D bioprinting refer both to the printing process, particularly noting printability, which is affected by physicochemical parameters such as rheological properties (viscosity, pseudo plasticity, viscoelasticity and yield strength) and cross-linking mechanisms; and those characteristics which allow them to be used as a biomedical material, such as biocompatibility, noting that the degradation kinetics thereof must coincide with the ability of cells to form their own extracellular matrix, and the degradation products thereof cannot be toxic for the same.

There is a wide range of materials used as bio-inks, among which are those that have been used over time in tissue engineering, having a hydrogel structure. These hydrogels used as bio-inks are divided into natural hydrogels (for example: alginate, gelatin, agarose, hyaluronic acid, chitosan, decellularized extracellular matrix, DNA peptides, and structural proteins such as collagen, silk fibroin and fibrin), and synthetic hydrogels (for example: poly(lactic-co-glycolic acid) (PLGA), pluronic acid, polyethylene glycol (PEG), poly(L-lactic acid) (PLA) and poly($\varepsilon$-caprolactone) (PCL)). Despite the variety of existing bio-inks, based on natural biopolymers, synthetic biopolymers and even mixtures of both, there are still many drawbacks limiting their use, especially with respect to the mechanical and structural properties of printed matrices, by showing in certain cases high hydrophilicity, which limits their use with cells, high viscosity, which hinders their printing, low shape integrity, rapid gelling and cross-linking, etc. Another drawback that has not yet been overcome also lies in the lack of biocompatibility or bioactive domains which allow cellular interaction.

To solve the aforementioned drawbacks of polymer-based bio-inks, several strategies have arisen: (1) Gelation methods stabilizing structures, primarily based on physical processes (ionic interactions, hydrogen bonds or hydrophobic interactions), chemical processes (covalent bonds generated by means of chemical reactions) or a combination of both (Jungst, T., et al. Chemical Reviews. 2016 116(3): 1496-1539); (2) use of multicomponent or hybrid inks, which usually attempt to make up for the structural deficiencies of one ink with the good printability of another ink, with there being mixtures in the state of the art of almost all existing bio-inks (Chimene, D., et al. Ann Biomed Eng. 2016, 44(6): 2090-2102.; (3) use of sacrificial inks to provide greater structure and support for printing and (4) use of synthetic materials as a support.

To date, there are several commercially available bio-ink compositions, such as: Gel4Cell based on gelatin polymers and combined with different growth factors (Bioink Solutions, Inc.), CELLINK based on nanocellulose and alginate (CELLINK), Bioink and Osteoink, based on PEG/gelatin/hyaluronic acid and calcium phosphate, respectively (RegenHU), and Bio127 and bioGel based on Pluronic F127 and methacrylated gelatin, respectively (Biobot). The drawback of bio-inks of this type lies in the fact that it is impossible to ensure their behavior in each production batch, since the gelatin polymers forming them are obtained from animal collagen hydrolysis and therefore their similarity cannot be ensured.

Although great progress has been made in the development of new bio-inks and their bioprinting techniques, there is still a need to find new biomaterials which fulfill all the necessary requirements for a suitable bio-ink, such as good printability and shape fidelity. Specifically, it would be desirable to develop new biomaterials with rapid gelling or solidification capacities providing a protective environment for printing with cells, as well as biomaterials which can be used at low concentrations, generating suitable biomechanical properties and high porosity.

## DESCRIPTION OF THE INVENTION

The present invention describes new ELR recombinant biopolymers formed by monomers having domains present in natural elastin in addition to monomers comprising the sequence called "silk" and/or monomers comprising the sequence called HLF belonging to a natural class of proteins called *zippers,* which are non-toxic and therefore suitable for use as bio-inks. Specifically, the present invention relates to compositions comprising said biopolymers, and mainly useful as bio-ink for 3D printing.

The ELR-based biomaterials have proven to have great potential in tissue engineering, fundamentally due to the fact that they are characterized by their extraordinary biocompatibility, biodegradability and adjustable mechanical properties; they can also generate a wide range of self-assembled structures, such as micelles, nanoparticles, hydrogels, membranes or nanofibers. Said polymers are used today in a wide range of biomedical applications such as: gene therapy, vaccine release systems, surface biofunctionalization and as hydrogels for tissue engineering applications. ELRs allow cell growth and proliferation and do not induce any immune response in biological systems, being, therefore, susceptible to being implanted. In the field of the tissue engineering, these materials have been used for the regeneration of cartilage and intervertebral disc, vascular grafts and eye and liver tissues.

Due to their production by means of recombinant DNA technology, the amino acid structure of ELRs can be designed to modulate their cross-linking or self-assembly capacity, wherein they are capable of self-assembling into physical and/or chemical hydrogels, with the subsequent improvement in mechanical properties, and this property will be used to favor their use as bio-inks in a 3D bioprinter. In addition, other functions can be implemented in their sequence by means of the fusion of other proteins or the inclusion of bioactive domains, such as cell adhesion motifs (RGD, REDV, among others), growth factors or metalloproteinases to favor disaggregation of the final structure.

Besides the biological requirements, the ELR biopolymers described in this invention have other particularities that can be employed for their use as bio-inks. These materials show a temperature response capacity that can be exploited for 3D bioprinting: due to the rapid conformational change that the polymer undergoes above a certain temperature, the polymer is capable of maintaining a liquid state in the printer cartridge, rapidly changing to a gel state when it is deposited on a heated plate. It is injected, therefore, in a liquid state and with low viscosities, reducing the stress applied on the needle, which facilitates deposition and protects cells against the possible membrane rupture. This behavior, referred to as Inverse Temperature Transition (ITT), is characterized by a transition temperature, $T_t$, which depends on mean polymer polarity, which can be modulated by varying the amino acid composition present in the ELR biopolymer sequence. Thus, the compositions of the invention, comprising the biopolymers herein described, as a result of the ITT property, are made to transition from a hydrated and unordered state when they are below their $T_t$ temperature, to an ordered hydrophobic folding when that $T_t$ is exceeded. The use of this unique property allows the use of said compositions as bio-inks since they are capable of generating polymer filaments that can be deposited under a temperature-controlled extrusion system. Said filaments are deposited with high precision and allow the formation of structural matrices which show temporal and spatial shape fidelity, which allows complex structures to be designed, layer-by-layer, with high versatility, reliability and reproducibility.

The biopolymers that are part of the composition described in the present invention comprise different ELR monomers, together with monomers comprising gelling reinforcement sequences, preferably monomers comprising the sequence called HLF which belongs to a natural class of proteins called *zippers,* and monomers comprising structure reinforcement sequences, preferably monomers comprising the sequence called "silk", originating from silkworms.

The ELR monomers used in the present invention for obtaining the described biopolymers are all based on the use of the same elastin domain VPGXG (SEQ ID NO: 7), wherein X can be any amino acid except the amino acid L-proline, with the amino acids valine, glutamic acid and isoleucine being preferred. Monomers B are among the ELR monomers used in the present invention.

Monomer B is hydrophilic and has a composition designed to not transition in the range of the physiological temperatures. For purposes of the present invention, monomer B comprises repeats of pentapeptide VPGXG (SEQ ID NO: 7), more specifically, monomer B comprises repeats of pentapeptides VPGVG (SEQ ID NO: 7) and VPGEG (SEQ ID NO: 7), even more specifically, monomer B comprises the sequence SEQ ID NO: 2 ([(VPGVG)$_2$(VPGEG)(VPGVG)$_2$].

Monomer C is hydrophobic and has a composition designed to produce a transition and cause an initial physical cross-linking at temperatures below the physiological temperature. For purposes of the present invention, monomer C comprises repeats of pentapeptide VGIPG (SEQ ID NO: 3), more specifically monomer C in the biopolymers of the invention comprises from 2 to 250 repeats of SEQ ID NO: 3, more specifically from 40 to 80 repeats of SEQ ID NO: 3, even more specifically, it comprises 60 repeats of the sequence SEQ ID NO: 3.

Monomer Y is among the monomers used to improve gelling of the biopolymer of the invention, said monomer comprising a combination of the amphiphilic polymer sequence of the elastin domain and the sequence called "silk" originating from the silkworm *Bombyx mori* (SEQ ID NO: 8; GAGAGS). For purposes of the present invention, monomer Y comprises repeats of the amino acid sequence SEQ ID NO: 8, more preferably, monomer Y comprises 1 to 15 repeats of SEQ ID NO: 8, more preferably 5 repeats. In another more preferred embodiment, monomer Y comprises the amino acid sequence as defined in SEQ ID NO: 5.

Monomer X is among the monomers used to improve the structure of the biopolymer of the invention, said monomer comprising reinforcement sequences for the biopolymers of the invention. Said monomer X comprises the amino acid sequence of the *"zipper"* structural motif, which is an amino acid sequence known as HLF and which belongs to a natural class of proteins called *zippers,* preferably said zipper motif belongs to the class of natural human zipper proteins. For purposes of the present invention, monomer X comprises an amino acid sequence as defined in SEQ ID NO: 4.

The biopolymers described in the present invention therefore comprise different repeats of the monomers described above to give rise to biopolymers useful as bio-inks for 2D and/or 3D printing.

Thus, in a first aspect, the present invention relates to a composition comprising a biopolymer comprising the amino acid sequences forming monomer B, monomer C, monomer X and monomer Y, or at least two biopolymers comprising monomers B, C and X and monomers B, C and Y, respectively, wherein

B comprises repeats of pentapeptide VPGXG (SEQ ID NO: 7), preferably B is an amino acid sequence comprising SEQ ID NO: 2,
C is an amino acid sequence comprising SEQ ID NO: 3,
X is an amino acid sequence comprising SEQ ID NO: 4, and
Y comprises 1 to 15 repeats of SEQ ID NO: 8, preferably Y is an amino acid sequence comprising SEQ ID NO: 5.

As shown in the examples included herein, the composition of the invention comprising at least one recombinant biopolymer formed by the monomers described above is useful for use as a bio-ink when said composition preferably comprises a biopolymer which comprises in its sequence, in addition to the ELR monomers (B and C), a combination of monomers X and Y, or even a combination of biopolymers wherein the first biopolymer comprises in its sequence, in addition to the ELR monomers (B and C), monomer X, and the second biopolymer comprises in its sequence, in addition to the ELR monomers (B and C), monomer Y. Thus, the inclusion in the biopolymer of monomer X comprising the zipper domain together with the rest of the monomers causes the amphiphilic interactions of the physical hydrogel formed to stabilize by means of the formation of coiled-coil interactions originating from the zipper of the sequence of the *zipper* domain. This phenomenon is observed physically, given that the transition of ELR monomers with temperature is accelerated and reinforced as a result of said *"zipper"* interactions; therefore, when the composition of the invention is used as a bio-ink, it shows good printability in the form of depositable filaments and good short-term stability. In contrast, as can be observed in the examples (see Example 4), if the composition exclusively comprises a biopolymer comprising, in addition to the ELR monomers, monomer X, said stability cannot be maintained over time, due to the reversibility of the interactions.

Moreover, the inclusion of monomer Y comprising the *silk* sequence together with the rest of the monomers forming the composition of the invention form a biopolymer in the form of a hydrogel through amphiphilic physical cross-linking, and wherein said biopolymer is stabilized through the formation of $\beta$ sheets originating from the *silk* sequence. This composition comprising ELR monomers and monomer Y, without including monomer X, does not allow reliable printing to be performed, given that the extrusion thereof through the syringe of the 3D printer is not homogeneous, does not form filaments and, therefore, does not hold its shape once printed (see Example 4). In contrast, said composition, as observed in Example 4, shows high stability over time. In this case, the transition occurring through the ITT mechanism is not rapid enough for the deposited polymer to maintain its structure, since the reinforcing interactions are slower and therefore effective in a step after the printing step.

Taking the above into account, the composition of the invention, therefore, will comprise a biopolymer comprising monomers B, C, X and Y, or alternatively a combination of biopolymers wherein the first biopolymer comprises monomers B, C and X and the second biopolymer comprises monomers B, C and Y.

Taking the aforementioned into account, the present invention is based in the following pillars:

- Combination of monomers comprising ELR sequences (monomers B) and monomers C together with monomers comprising *silk* (monomer Y) and *zipper* (X monomer) sequences; or combinations of biopolymers comprising ELR monomers together with *silk* monomers (monomer Y), and biopolymers comprising ELR monomers (monomers B) and monomers C together with *zipper* monomers (monomer X). As such, monomers X provide the necessary printability, that is, the capacity to initially maintain the structure, while monomers Y ensure that the structure is maintained over time.
- Concentrations of the biopolymers (see Example 3) forming the composition of the invention allow them to be dissolved in several solvents, showing low viscosity and Newtonian behavior (see Example 4), thereby facilitating printing in 3D printers, avoiding the application of high printing forces which may damage both the printer and the materials in solution together with the bio-ink, as well as facilitating the use of needles having smaller diameters, allowing finer filaments to be formed.

- The compositions of the invention allow being used in 2D and/or 3D printing with very good shape fidelity (see Examples 4 and 6). They furthermore allow being used for printing supports and encapsulating a wide range of active ingredients and cells, therefore being useful in biomedicine, for example, but not limited to, regenerative medicine, for example, for *in vitro* or *in vivo* cell growth in cell therapy methods for tissue regeneration. To this end, the cells and/or active ingredients are preferably homogeneously dispersed in the compositions of the invention, such that after printing, they are distributed in a predetermined manner in the matrix and allow a controlled release of the active ingredient or good adhesion and proliferation of the cells, regenerating damaged tissues and acting, therefore, as an effective implant and as a natural extracellular matrix.

- The deposition of these compositions as bio-inks on the printing surface is performed in a controlled manner through a design previously stipulated by the inventor through specific software. The printing process requires keeping the extrusion head at a low temperature, below the Tt of the composition, while the temperature of the heating bed is kept above the Tt of the composition. As such, the composition in solution of the syringe transitions right when it is deposited, such that it goes from a disorganized liquid state when it is in the needle to an ordered, hydrophobic hydration state when it is dispensed on the bed, thus achieving the formation of filaments on the bed, which can be deposited layer-by-layer, maintaining the structure.

- In addition, the composition of the invention may further comprise at least another monomer D, wherein said monomer D comprises sequences of bioactive domains, such as for example RGD and REDV cell adhesion motifs, growth factors such as VEGF, or metalloproteinases which favor the controlled disaggregation of the formed structures, etc. Introducing bioactive domains of this type in biopolymers comprising the composition of the invention allows designing structures with different functionalities and bioactivities, aimed at the inclusion of, for example, specific cell binding sequences, recombinantly introduced in the sequence thereof, where this possibility does not exist in any bio-ink existing today. The introduction of different sequences will predetermine, for example, the cellular behavior on structures, being able to generate matrices with well-differentiated cellular areas, which is a necessary property for the mimetic design of tissues or micro-organs that can be implanted.

Therefore, in a first aspect, the present invention relates to a composition comprising a biopolymer comprising monomers B, C, X and Y, or at least two biopolymers comprising monomers B, C and X and monomers B, C and Y, respectively, wherein

B is an amino acid sequence made of ELR domain repeats that comprises repeats of pentapeptide VPGXG (SEQ ID NO: 7), preferably B is an amino acid sequence comprising SEQ ID NO: 2,
C is an amino acid sequence made of ELR domain repeats and comprises the SEQ ID NO: 3,
X is an amino acid sequence comprising the *"zipper"* structural motif and comprises the SEQ ID NO: 4, and
Y is an amino acid sequence comprising a combination of the elastin domain sequence and the sequence called "silk" originating from the silkworm *Bombyx mori* (SEQ ID NO: 8; GAGAGS) and comprises 1 to 15 repeats of SEQ ID NO: 8, preferably Y is an amino acid sequence comprising SEQ ID NO: 5.

In another preferred embodiment, the composition of the invention further comprises monomer D. Monomer D is a cell binding sequence comprising, preferably, at least one peptide selected from the list consisting of: RGD (Arg-Gly-Asp), as a cell adhesion domain of the $\alpha v\beta 3$, $\alpha 5\beta 1$ and $\alpha IIb\beta 3$ integrin receptor (SEQ ID NO: 9), LDT (SEQ ID NO: 27), SEQ ID NO: 10, SEQ ID NO: 17, SEQ ID NO: 18 or SEQ ID NO: 19, or a heparin-binding domain or a sugar-binding domain derived from lectin, agglutinin, growth factors, metalloproteinases, in addition to the GTAR sequence (SEQ ID NO: 28) and DRIR sequence (SEQ ID NO: 29), which belong to the enzyme, uPA (urokinase plasminogen activator system) and other similar sequences which favor protein degradation. Preferably, monomer D comprises the RGD domain (SEQ ID NO: 9) and is preferably SEQ ID NO: 6.

The RGD domain is well known and consists, as its name indicates, of the amino acids arginine, glycine and aspartic acid. This domain is recognized by cell surface proteins of various cell types and functions as a cell adhesion domain. The REDV domain (SEQ ID NO: 10), which is also well known and consists, as its name indicates, of the amino acids arginine, glutamic acid, aspartic acid and valine also functions as a cell adhesion domain and is recognized by endothelial cells. A heparin-binding domain functions as a cell-binding domain since it is a cell surface glycosaminoglycan-binding domain. Likewise, a sugar-binding domain allows the binding to cells through the sugars having the membrane glycoproteins. Lectin and agglutinin have well-known sugar-binding domains. SEQ ID NO: 18 is present in laminin and is recognized by various cell types, SEQ ID NO: 19 is recognized by neurites, in other words, any expansion of the soma of a neuron, whether a dendrite or an axon. These sequences that are part of the biopolymer of the invention are recognized by their respective cell types and promote the binding thereof. The biopolymers containing SEQ ID NO: 10 or SEQ ID NO: 19 can be used in tissue generation.

Additionally, the biopolymers of the invention may optionally comprise an additional monomer, monomer A, which can be attached to their 5' end and is the result of the transcription of a starting nucleotide sequence. Thus, monomer A can comprise SEQ ID NO: 20, which is the result of the transcription of the nucleotide sequence SEQ ID NO: 1.

Amino acid sequences (the term "peptides" can be used interchangeably to refer to the amino acid sequences) forming the monomers according to the described structures giving rise to the biopolymers of the invention can be attached by a covalent bond or any other type of bond giving rise to a structure maintaining the properties of the biopolymers of the present invention. The bond is selected from, but not limited to, the list comprising hydrogen bonds, ion pairing, hydrophobic association or the formation of inclusion complexes.

In a preferred embodiment, the monomers that are part of the biopolymers of the invention can be attached to one another directly, or by means of sequences facilitating their attachment referred to as polypeptide spacers or linkers.

Thus, for the purposes of the present invention, the term "linker" or "polypeptide spacer" refers to a short amino acid sequence, preferably of up to 20 amino acids in length, more preferably, of up to 15 amino acids in length, more preferably of up to 10 amino acids in length, and even more preferably, of up to 5 amino acids in length, situated between the amino acid sequences of monomers B, C, X, Y and/or D forming the biopolymers of the invention as generally described or in formulas (I) or (II) defined below, allowing the attachment between the different monomers. Advantageously, said polypeptide spacer is a structurally flexible peptide, such as a peptide that gives rise to a non-structured domain. Virtually any structurally flexible peptide can be used as a peptide spacer; however, illustrative, non-limiting examples of said peptide spacers include peptides containing amino acid residue repeats, e.g., of Val, Gly and/or Ser, or any other suitable amino acid residue repeat.

In another preferred embodiment, the composition of the invention is characterized in that the biopolymer comprised in it has structure (I):

$$[(B_b\text{-}C_c)\text{-}Z_z]_n\text{-}D_d$$

wherein B, C and D are the monomers described above,

Z is selected from monomers X and/or Y defined above,

b has values of between 5 and 15,

c has values of between 50 and 70;

z has values of between 1 and 5

n has values of between 1 and 5,

d has values of between 0 and 3, and

it is characterized in that it comprises a first biopolymer of structure (I), wherein Z is SEQ ID NO: 4, and a second biopolymer of structure (I), wherein Z is SEQ ID NO: 5, preferably wherein the first biopolymer is found in the composition at a concentration of between 40-60 % by weight and the second biopolymer is found in the composition at a concentration of between 60-40 % by weight.

In another preferred embodiment of the composition of the invention, said composition is characterized in that the biopolymer comprised in it has structure (II):

$$Z1_z\text{-}[(B_b\text{-}C_c)\text{-}Z2_z]_n\text{-}D_d,$$

wherein B, C and D have been previously defined,

Z1 is an amino acid sequence comprising the *"zipper"* structural motif, more preferably, it comprises monomer X, even more preferably it comprises SEQ ID NO: 4 or SEQ ID NO: 5, and

Z2 is an amino acid sequence comprising the "silk" structural motif, more preferably, it comprises monomer Y, even more preferably it comprises SEQ ID NO: 5 or SEQ ID NO: 4,

respectively; preferably Z1 is amino acid sequence SEQ ID NO: 4 and Z2 is amino acid sequence SEQ ID NO: 5;

b, c, z, n and d have been previously defined.

In another preferred embodiment of the composition of the invention, said composition is characterized in that b has a value of 10, c has a value of 60, z has a value of 1, n has a value of 2 and d has a value of 0 or 1.

In another preferred embodiment of the composition of the invention, the biopolymer is selected from SEQ ID NO: 16 or from the combination of biopolymers of SEQ ID NO: 14 and SEQ ID NO: 15

In another more preferred embodiment, the composition of the invention comprises the biopolymer of SEQ ID NO: 16.

In another preferred embodiment, the composition of the invention may further comprise cells, bioactive molecules, active ingredients or combinations thereof.

Table 1 shows the different biopolymers described in the present description, together with each of the monomers comprising them and the structure of each one.

Table 1. Characterization of the biopolymers described herein.

| Name | Structure | Sequence | SEQ ID NO: |
|---|---|---|---|
| **Polymer 2** (101696 Da) | $\{(B_{10}\text{-}C_{60})\text{-}Y\}_2$ | $\{([(VPGVG)_2\text{-}(VPGEG)\text{-}(VPGVG)_2]_{10}[VGIPG]_{60}\text{-}[V(GAGAGS)_5G]_2\}_2$ | 12 |
| **Polymer 3** (104119 Da) | $\{(B_{10}\text{-}C_{60})\text{-}X\}_2$ | $\{[VPGVG)_2\text{-}(VPGEG)\text{-}(VPGVG)_2]_{10}[VGIPG]_{60}\text{-}[VGGGGGKENQIAIRASFLEKENSALRQEVADLRKELGKCKNILAKYEAGGGGG]\}_2$ | 13 |
| **Polymer 4** (123345 Da) | $\{(B_{10}\text{-}C_{60})\text{-}X\}_2\text{-}D$ | $\{[VPGVG)_2\text{-}(VPGEG)\text{-}(VPGVG)_2]_{10}[VGIPG]_{60}\text{-}[VGGGGGKENQIAIRASFLEKENSALRQEVADLRKELGKCKNILAKYEAGGGGG]\}_2\text{-}([VPGIG]_5AVTGRGDSPASS)_6\text{-}V$ | 14 |
| **Polymer 5** (120921 Da) | $\{(B_{10}\text{-}C_{60})\text{-}Y\}_2\text{-}D$ | $\{([(VPGVG)_2\text{-}(VPGEG)\text{-}(VPGVG)_2]_{10}[VGIPG]_{60}\text{-}[V(GAGAGS)_5G]_2\}_2\text{-}([VPGIG]_5AVTGRGDSPASS)_6\text{-}V$ | 15 |
| **Polymer 6** (126393 Da) | $X\text{-}\{(B_{10}\text{-}C_{60})\text{-}Y\}_2\text{-}D$ | $[VGGGGGKENQIAIRASFLEKENSALRQEVADLRKELGKCKNILAKYEAGGGGG]\text{-}\{([(VPGVG)_2\text{-}(VPGEG)\text{-}(VPGVG)_2]_{10}[VGIPG]_{60}\text{-}[V(GAGAGS)_5G]_2\}_2\text{-}([VPGIG]_5AVTGRGDSPASS)_6\text{-}V$ | 16 |

A second aspect of the present description relates to a nucleic acid comprising a nucleotide sequence coding for the amino acid sequence of the biopolymer of the first aspect of the invention.

The nucleic acid includes nucleic acid sequences, the transcription product of which, messenger RNA (mRNA), codes for the same amino acid sequence (hereinafter, amino acid sequence of the present invention or amino acid sequence of the invention). Degenerate variant sequences of the nucleotide sequences, the product of which is a biopolymer with the same characteristics as the biopolymer of the invention, are also included. Nucleotide sequences coding for amino acid sequences with modifications at their N-terminal end, C-terminal end and/or at any internal amino acid position such that the function of the resulting biopolymer is the same as the function resulting from the translation of the sequence of mRNA transcribed from the nucleotide sequence, are also included. The amino acid sequence can be coded by any nucleotide sequence giving rise to any of the amino acid sequences of the invention. Given that the genetic code is degenerate, one same amino acid can be coded by different codons (triplets); to that end, the same amino acid sequence can be coded by different nucleotide sequences.

As mentioned earlier, the nucleotide sequences coding for monomers B, C, X, Y, and/or D comprised in the biopolymers of the invention may be attached to one another directly, or by means of polypeptide spacers or linkers. For purposes of the present invention, the polynucleotide sequence coding for each of the biopolymers of the invention may therefore comprise linkers.

In a preferred embodiment, the nucleotide sequences coding for each of the biopolymers described herein are selected from the list consisting of: SEQ ID NO: 21, which codes for biopolymer 1 comprising SEQ ID NO: 11; SEQ ID NO: 22, which codes for biopolymer 2 comprising SEQ ID NO: 12; SEQ ID NO: 23, which codes for biopolymer 3 comprising SEQ ID NO: 13; SEQ ID NO: 24, which codes for biopolymer 4 comprising SEQ ID NO: 14; SEQ ID NO: 25, which codes for biopolymer 5 comprising SEQ ID NO: 15; and SEQ ID NO: 26, which codes for biopolymer 6 comprising SEQ ID NO: 16.

The nucleic acid may have a nucleotide sequence attached to its 5' end serving as a transcription start sequence. The sequence may be, but is not limited to, the nucleotide sequence SEQ ID NO: 1, which codes in each biopolymer of the invention for the amino acid sequence SEQ ID NO: 20, also called monomer A in the final structure of each biopolymer. Likewise, the nucleic acid may have a transcription termination sequence attached to its 3' end, such as, but not limited to, sequence GTATGA.

The nucleotide sequence coding for the amino acid sequence of the biopolymers that are part of the composition of the present invention is inserted in an expression vector. Thus, a further aspect of the present description relates to an expression vector comprising the nucleic acid described above.

For purposes of the present invention, the term "expression vector" refers to a DNA fragment having the capacity to replicate in a certain host and, as the term indicates, it can serve as a vehicle for multiplying another DNA fragment that has been fused thereto (insert). Insert refers to a DNA fragment fused to the vector; in the case of the present invention, the vector can comprise any of the nucleotide sequences coding for any of the biopolymers of the invention, fused thereto, which can replicate in a suitable host. The vectors can be plasmids, cosmids, bacteriophages or viral vectors, without excluding another type of vectors corresponding to the provided definition of vector.

The transfection of a cell, defined in an earlier paragraph, is carried out with techniques known in the state of the art, for example, but not limited to, electroporation, biolistics, *Agrobacterium tumefaciens* or any other technique which allows integration of any of the nucleic acids in the DNA of the host cell, whether genomic, chloroplastic or mitochondrial.

The expression of the nucleic acid in the cell gives rise to a biopolymer which can be purified by means of techniques known in the state of the art, as discussed above.

A third aspect of the present description relates to an isolated cell transfected with the nucleic acid described above.

The term "cell" as it is understood in the present description refers to a prokaryotic or eukaryotic cell. The cell can be a bacterium capable of replicating transformed foreign DNA, such as for example any of the strains of the species *Escherichia coli* or a bacterium capable of transferring the DNA of interest into a plant, such as for example *Agrobacterium tumefaciens.* Preferably, the cell refers to a plant eukaryotic cell, and within this group, more preferably, to those cells belonging to the kingdom Plantae. Thus, if the cell is a plant cell, the term cell comprises at least a cell of the parenchyma, a meristem cell or a cell of any type, differentiated or undifferentiated. Likewise, a protoplast (a plant cell lacking a cell wall) is also included in this definition.

The term "transfection" refers to the introduction of external genetic material into cells by means of plasmids, viral vectors (in this case transduction can also be mentioned) or other transfer tools. The term transfection for non-viral methods is used in reference to mammalian eukaryotic cells, while the term transformation is preferred for describing non-viral transfers of genetic material into bacteria and non-animal eukaryotic cells such as fungi, algae or plants.

Once their sequence has been established, the biopolymers comprising the composition of the invention can be the object of additional treatments such as homogenization and purification processes, widely known in the state of the art, which help to obtain the desired level of cytocompatibility, allowing the combined use thereof with cells or other bioactive molecules and/or components with different diagnostic activities, and even in combination with compositions used as bio-inks, such as for example natural bio-inks based on alginate, gelatin, agarose, hyaluronic acid, chitosan, decellularized extracellular matrix, DNA peptides, and structural proteins such as collagen, silk fibroin and fibrin; synthetic bio-inks such as for example poly(lactic-co-glycolic acid) (PLGA), pluronic acid, polyethylene glycol (PEG), poly(L-lactic acid) (PLA) and poly(ε-caprolactone) (PCL). Likewise, they can be processed through different mechanical, enzymatic and/or chemical steps for achieving the desired polymer properties, both morphological and physical properties. Several characterization processes will also be used to ensure their use in perfect conditions, such as the analysis of their amino acid composition, physical characterizations or the rheological analysis of the bio-inks formed by the compositions described in the present invention.

Another aspect of the present invention relates to the use of the composition of the invention as a bio-ink, preferably as a bio-ink for the 2D or 3D printing of biomaterials.

Another aspect of the invention refers to the use of the composition of the invention for the preparation of a support for obtaining organs and/or tissues, or for the encapsulation of active ingredients or cells

Another aspect of the present invention relates to a bio-ink comprising the composition as described in the present invention.

For purposes of the present invention, the bio-inks are prepared using sterile components and always ensuring their use under conditions of sterility. The different biopolymers designed for forming the described bio-inks can be printed with or without cells and can also be used as a support for other bio-inks, such as materials prepared from decellularized tissues and organs.

Another aspect of the present invention relates to a 3D or 2D biomaterial comprising the composition of the invention.

In any of the embodiments described herein, the compositions, bio-ink and biomaterials described in the invention can further include one or more agents (for example, excipients, additives, active ingredients, biologically active agents, etc.) suitable for the intended purposes, including therapeutic agents (for example, biologically active agents) and biological samples. Typically, the addition of such agents is said to "functionalize" the composition, bio-ink or biomaterial, providing added functionality. Non-limiting examples of said agents suitable for being added for functionalization of the compositions, bio-inks and biomaterials of the invention include, but are not limited to: conductive or metallic particles; inorganic particles; dyes/pigments; drugs or active ingredients (for example, antibiotics, small molecules or low molecular weight organic compounds); proteins and fragments or complexes thereof (for example, enzymes, antigens, antibodies and antigen-binding fragments thereof); cells and fractions thereof (viruses and viral particles, prokaryotic cells such as bacteria, eukaryotic cells such as mammalian cells and plant cells, fungi).

As it is used herein, the term "biologically active agent" refers to any molecule exercising at least one *in vitro* or *in vivo biological effect.* For example, the biologically active agent can be a therapeutic agent for treating or preventing a disease state or condition in a subject. Biologically active agents include, but are not limited to, organic molecules, inorganic materials, proteins, peptides, nucleic acids (for example, genes, gene fragments, gene regulatory sequences and antisense molecules), nucleoproteins, polysaccharides, glycoproteins and lipoproteins. The classes of biologically active compounds that may be incorporated in the composition described herein include, but are not limited to, anticancer agents, antibiotics, analgesics, anti-inflammatory agents, immunosuppressants, enzyme inhibitors, antihistamines, anticonvulsants, hormones, muscle relaxers, antispasmodics, ophthalmic agents, prostaglandins, antidepressants, antipsychotics, trophic factors, osteoinductive proteins, growth factors and vaccines.

In some embodiments, the additive is a therapeutic agent. As it is used herein, the term "therapeutic agent" means a molecule, group of molecules, complex or substance administered to an organism for diagnostic, therapeutic, preventive medical or veterinary purposes. As it is used herein, the term "therapeutic agent" includes a "drug" or a "vaccine". This term can also specifically include nucleic acids and compounds comprising nucleic acids producing a therapeutic effect.

The term "therapeutic agent" also includes an agent which is capable of providing a local or systemic biological, physiological or therapeutic effect in the biological system to which it is applied. For example, the therapeutic agent can act to control infection or inflammation, potentiate cell growth and tissue regeneration, control tumor growth, act as an analgesic, promote anti-cell binding and potentiate bone growth, among other functions. Other suitable therapeutic agents can include antiviral agents, hormones, antibodies or therapeutic proteins. Other therapeutic agents include prodrugs, which are agents that are not biologically active when administered but, after administration to a subject, are converted into biologically active agents through metabolism or some other mechanism. Additionally, a silk-based drug delivery composition may contain a therapeutic agent or combinations of two or more therapeutic agents.

In some embodiments, the agent stimulates tissue formation, and/or natural tissue healing and re-growth, and any combination thereof. Agents increasing new tissue formation and/or stimulating native tissue healing or re-growth at the injection site can include, among others, growth factors (fibroblast growth factor (FGF), transforming growth factor beta (TGF-beta, platelets). derived growth factor (PDGF), epidermal growth factors (EGF), connective tissue-activated peptides (CTAP), osteogenic factors including bone morphogenetic proteins, heparin, angiotensin II (A-II) and fragments thereof, insulin-like growth factors, tumor necrosis factors, interleukins, colony stimulating factors, erythropoietin, nerve growth factors, interferons, biologically active analogs, fragments and derivatives of such growth factors, and any combination thereof.

In some embodiments, the agent is a wound healing agent. As it is used herein, a "wound healing agent" is a compound or composition which actively promotes the wound healing process.

In certain embodiments, the active agents described herein are immunogens. In one embodiment, the immunogen is a vaccine.

In some embodiments, the agent can be a cell, for example, a biological cell. The cells useful for the incorporation in the composition can come from any source, for example, mammal, insect, plant, etc. In some embodiments, the cell can be a human cell, primate cells, mammalian cells, rodent cells, etc., preferably a human cell. In some embodiments, the cell can be a genetically modified cell. A cell can be genetically modified to express and secrete a desired compound, for example, a bioactive agent, a growth factor, a differentiation factor, cytokines and the like. The methods for genetically modifying cells to express and secrete compounds of interest are known in the art and can be readily adapted by a person skilled in the art.

In some embodiments, the compositions, bio-inks and biomaterials of the invention can include a colorant, such as a pigment or dye or a combination thereof. Pigments and colorants which are organic and/or inorganic, fluorescent, etc., can be included.

Therefore, in view of that described above, another aspect of the present invention relates to the composition, bio-ink and biomaterial as described herein for use as a drug.

Another aspect of the present invention relates to the composition, bio-ink and biomaterial as described in the present invention for use in tissue regeneration, as well as for generating tissues mimicking pathologies, which serve as disease models, or which contain defects for the testing of new therapeutic and/or prophylactic compounds, thus preventing the use of animal models.

Another aspect of the present description relates to a method for obtaining the composition of the invention, comprising the following steps:

(a) culturing the cell described above under conditions suitable for the expression of the nucleic acid described above, and
(b) purifying the biopolymer coded by said nucleic acid.

The degree of compositional complexity imposed by multifunctional design needs cannot be achieved by standard macromolecular synthesis techniques. The biopolymer is obtained as a recombinant protein, by means of adapted molecular and biotechnological biology techniques, in genetically modified microorganisms or plants.

The nucleotide sequence coding for the amino acid sequence of the biopolymer of the present invention is inserted in an expression vector defined above.

The transfection of a cell, defined in an earlier paragraph, is carried out with techniques known in the state of the art, for example, but not limited to, electroporation, biolistics, *Agrobacterium tumefaciens* or any other technique which allows integration of any of the nucleic acids of the invention in the DNA of the host cell, whether genomic, chloroplastic or mitochondrial.

The expression of the nucleic acid in the cell gives rise to a biopolymer which can be purified by means of techniques known in the state of the art.

**BRIEF DESCRIPTION OF THE FIGURES**

**Figure 1** shows acrylamide gel electrophoresis of biopolymer 1 with the molecular weight marker in the lane on the left and biopolymer 1 in the lane on the right. The molecular weights are indicated in kilodaltons (kDa).

**Figure 2** shows a mass spectroscopy (MALDI-ToF, or *"Matrix-assisted laser desorption/ionization - time of flight"*) analysis of biopolymer 1 which shows the value of its experimental molecular mass of 92897 Da, wherein the theoretical molecular mass is 93175 Da and the difference between both can be attributed to the measurement error. The monodisperse nature of the molecule is also observed, with only one narrow peak being seen.

**Figure 3** shows an infrared spectroscopy (FTIR-ATR, or *"Fourier Transform Infrared - Attenuated Total Reflectance"*) analysis of biopolymer 1 which shows the characteristic signals of the amide groups (-1700 cm-1) present in the designed protein biopolymers.

**Figure 4** shows a nuclear magnetic resonance (NMR) analysis of biopolymer 1 in which the signal of the hydrogens belonging to the amine group NH (7.5 - 8.5 ppm), to the methyl group $CH_3$ (0.5 - 1.0 ppm) and to the methylene group CH (1.0 - 2.3; 3.5 - 4.5 ppm) is observed.

**Figure 5** shows acrylamide gel electrophoresis of biopolymer 2 with the molecular weight marker in the lane on the right and biopolymer 2 in the lane on the left. The molecular weights are indicated in kilodaltons (kDa).

**Figure 6** shows a MALDI-TOF analysis of biopolymer 2 which shows the value of its experimental molecular mass of 101664 Da, wherein the theoretical molecular mass is 101696 Da and the difference between both can be attributed to the measurement error.

**Figure 7** shows a FTIR-ATR analysis of biopolymer 2 which shows the characteristic signals of the amide groups (-1700 cm-1) present in the designed protein polymers.

**Figure 8** shows a nuclear magnetic resonance (NMR) analysis of biopolymer 2 in which the signal of the hydrogens belonging to the amine group NH (7.5 - 8.5 ppm), to the methyl group $CH_3$ (0.5 - 1.0 ppm) and to the methylene group CH (1.0 - 2.3; 3.5 - 4.5 ppm) is observed.

**Figure 9** shows acrylamide gel electrophoresis of biopolymer 3 with the molecular weight marker in the lane on the left and biopolymer 3 in the lane on the right. The molecular weights are indicated in kilodaltons (kDa).

**Figure 10** shows a MALDI-TOF analysis of biopolymer 3 which shows the value of its experimental molecular mass of 103793 Da, wherein the theoretical molecular mass is 104119 Da and the difference between both can be attributed to the measurement error.

**Figure 11** shows a FTIR-ATR analysis of biopolymer 3 which shows the characteristic signals of the amide groups (-1700 cm-1) present in the designed protein polymers.

**Figure 12** shows a nuclear magnetic resonance (NMR) analysis of biopolymer 3 in which the signal of the hydrogens belonging to the amine group NH (7.5 - 8.5 ppm), to the methyl group $CH_3$ (0.5 - 1.0 ppm) and to the methylene group CH (1.0 - 2.3; 3.5 - 4.5 ppm) is observed.

**Figure 13** shows acrylamide gel electrophoresis of biopolymer 4 with the molecular weight marker in the lane on the right and biopolymer 4 in the lane on the left. The molecular weights are indicated in kilodaltons (kDa).

**Figure 14** shows a MALDI-TOF analysis of biopolymer 4 which shows the value of its experimental molecular mass of 122882 Da, wherein the theoretical molecular mass is 123345 Da and the difference between both can be attributed to the measurement error.

**Figure 15** shows a FTIR-ATR analysis of biopolymer 4 which shows the characteristic signals of the amide groups (-1700 cm-1) present in the designed protein polymers.

**Figure 16** shows a nuclear magnetic resonance (NMR) analysis of biopolymer 4 in which the signal of the hydrogens belonging to the amine group NH (7.5 - 8.5 ppm), to the methyl group $CH_3$ (0.5 - 1.0 ppm) and to the methylene group CH (1.0 - 2.3; 3.5 - 4.5 ppm) is observed.

**Figure 17** shows acrylamide gel electrophoresis of biopolymer 5 with the molecular weight marker in the lane on the right and biopolymer 5 in the lane on the left. The molecular weights are indicated in kilodaltons (kDa).

**Figure 18** shows a MALDI-TOF analysis of biopolymer 5 which shows the value of its experimental molecular mass of 120611 Da, wherein the theoretical molecular mass is 120921 Da and the difference between both can be attributed to the measurement error.

**Figure 19** shows a FTIR-ATR analysis of biopolymer 5 which shows the characteristic signals of the amide groups (-1700 cm-1) present in the designed protein polymers.

**Figure 20** shows a nuclear magnetic resonance (NMR) analysis of biopolymer 5 in which the signal of the hydrogens belonging to the amine group NH (7.5 - 8.5 ppm), to the methyl group $CH_3$ (0.5 - 1.0 ppm) and to the methylene group CH (1.0 - 2.3; 3.5 - 4.5 ppm) is observed.

**Figure 21** shows acrylamide gel electrophoresis of biopolymer 6 with the molecular weight marker in the lane on the right and biopolymer 6 in the lane on the left. The molecular weights are indicated in kilodaltons (kDa).

**Figure 22** shows a MALDI-TOF analysis of biopolymer 6 which shows the value of its experimental molecular mass of 125857 Da, wherein the theoretical molecular mass is 126393 Da and the difference between both can be attributed to the measurement error.

**Figure 23** shows a FTIR-ATR analysis of biopolymer 6 which shows the characteristic signals of the amide groups (-1700 cm-1) present in the designed protein polymers.

**Figure 24** shows a nuclear magnetic resonance (NMR) analysis of biopolymer 6 in which the signal of the hydrogens belonging to the amine group NH (7.5 - 8.5 ppm), to the methyl group $CH_3$ (0.5 - 1.0 ppm) and to the methylene group CH (1.0 - 2.3; 3.5 - 4.5 ppm) is observed.

**Figure 25** shows photographs of the biomaterial printed with the composition comprising different concentrations (300, 250, 200, 180, 150 and 120 mg/ml) of pre-cured biopolymer 5 (SEQ ID NO: 15) shown in column A, and of biopolymer 4 (SEQ ID NO: 14) shown in column B, using PBS1x as solvent.

**Figure 26** shows photographs of different biomaterials printed with the different compositions of the invention at a concentration of 250 mg/ml using PBS1x as solvent, wherein the printability (Column A) and fibrillar observation (Column B) of said biomaterials are clearly shown. BP: Biopolymer. The percentage of biopolymer combinations refers to the percentage expressed by weight.

**Figure 27** shows viscosity (expressed in Pascals per second, Pa.s) of the bio-inks formed by different biopolymers of the invention subjected to an increasing shear velocity (1/s).

**Figure 28** shows an evaluation of the variation in viscosity in different bio-inks of the invention subjected to a high shear velocity for a short time interval. Step 1: Shear velocity of 5 s$^{-1}$. Step 2: Shear velocity of 1000 s$^{-1}$. Step 3: Shear velocity of 5 s$^{-1}$.

**Figure 29** shows the effect of temperature on the viscosity of different analyzed bio-inks of the invention.

**Figure 30** shows photographs of different structures printed with the bio-ink comprising biopolymer 4 (SEQ ID NO: 14) using PBS1x as solvent, where stability of the printed structures over 3 days is shown.

**Figure 31** shows photographs of different structures printed with the bio-ink comprising pre-cured biopolymer 5 (SEQ ID NO: 15) using PBS1x as solvent, where stability of the printed structures over 2 days is shown.

**Figure 32** shows photographs of different structures printed with the bio-ink comprising the combination of biopolymers, 60 % by weight of biopolymer 4 (SEQ ID NO: 14) and 40 % by weight of pre-cured biopolymer 5 (SEQ ID NO: 15) using PBS1x as solvent, where stability of the printed structures over 40 days is shown.

**Figure 33** shows photographs of different structures printed with the bio-ink comprising biopolymer 6 (SEQ ID NO: 16) using PBS1x as solvent, where stability of the printed structures over 40 days is shown.

**Figure 34** shows a graph showing an analysis of early cell adhesion at times of 30 min, 2 hours and 4 hours, of the mixtures of biopolymer 4 (60 % by weight) of SEQ ID NO: 14 and pre-cured biopolymer 5 (40 % by weight) of SEQ ID NO: 15, comprising the RGD adhesion sequence (white blocks), and of the mixture of biopolymer 3 (60 % by weight) of SEQ ID NO: 13 and biopolymer 2 (40 % by weight) of SEQ ID NO: 12, not comprising cell adhesion sequence (black blocks).

**Figure 35** shows an analysis of cell proliferation over 21 days on printed surfaces based on mixtures of biopolymer 4 (60 % by weight) of SEQ ID NO: 14 and pre-cured biopolymer 5 (40 % by weight) of SEQ ID NO: 15, comprising the RGD adhesion sequence (white blocks), and of the mixture of biopolymer 3 (60 % by weight) of SEQ ID NO: 13 and biopolymer 2 (40 % by weight) of SEQ ID NO: 12, not comprising cell adhesion sequence (black blocks).

**Figure 36** shows a microscopic photograph of a surface printed with the bio-ink comprising the combination of biopolymer 4 (60 % by weight) of SEQ ID NO: 14 and pre-cured biopolymer 5 (40 % by weight) of SEQ ID NO: 15 using PBS1x as solvent, on which HFF-1 cells have been seeded and cultured for 7 days.

**Figure 37** shows microscopic photographs of a surface printed with the bio-ink comprising the combination of biopolymer 4 (60 % by weight) of SEQ ID NO: 14 and pre-cured biopolymer 5 (40 % by weight) of SEQ ID NO: 15 using PBS1x as solvent, on which HFF-1 cells have been seeded and cultured for 7 days. Different focal planes are shown to corroborate the three-dimensionality of the system.

**Figure 38** shows the viability of human fibroblasts HFF-1 printed together with biopolymer 6 over 21 days. Viability of the printed gratings is compared together with the control (non-printed deposited material).

**Figure 39** shows microscopic photographs of a surface printed with biopolymer 6 mixed with human fibroblasts HFF-1 over 21 days. The scale corresponds to 500 $\mu$m.

## EXAMPLES

The invention is illustrated below by means of assays conducted by the inventors, describing the synthesis of the composition of the invention, as well as its features. The examples are provided for the purpose of understanding the description and are not intended to limit the present invention.

### EXAMPLE 1. Obtaining and characterization of the recombinant protein biopolymers used in the assays described herein.

The design and obtaining of synthetic nucleotide sequences coding for the amino acid sequences of the different biopolymers used, including the biopolymers comprising the composition of the invention, was performed as described in WO/2010/092224. Likewise, the expression, purification and characterization of the biopolymers was carried out as described in WO/2010/092224.

Briefly, the ELRs are designed and produced by means of recombinant DNA technologies. Once the nucleotide sequence coding for the desired protein has been introduced in bacterial strain *Escherichia coli,* said strain is subjected to culturing in a fermenter, which allows absolute control of the production conditions. When the stationary phase in the bacterial culture growth curve is achieved, the desired ELR is extracted by means of ultrasonic lysis of the bacterial wall. Purification of the biopolymer will be carried out using its inverse temperature transition property, performing bacterial debris heating and cooling cycles until obtaining the pure polymer.

After a salt elimination process through dialysis, all the biopolymers used are lyophilized, showing a whitish and cottony appearance, and are set aside until they are used in this state at -20 °C.

To characterize the obtained biopolymers, the following techniques are used:

- Acrylamide gel electrophoresis (PAGE) in the presence of SDS which allows the molecular weight of the biopolymer to be approximately estimated, in addition to the purity thereof to be verified.

- MALDI-TOF mass spectrometry in a Q-Star spectrometer to precisely obtain the molecular weight of the polymer.
- Proton nuclear magnetic resonance (H1-NMR) spectrum in a Bruker ARX300 spectrometer.
- Infrared (FT-IR) spectrum using a Cary 50 spectrophotometer.
- HPLC chromatography with UV detection using a WATERS 600 HPLC gradient system with a WATERS 2487 detector, which allows the amino acid composition to be determined.
- Differential scanning calorimetry (DSC) of aqueous solutions of the material with a concentration of 50 mg/ml in Mettler Toledo 822e DSC equipment, to obtain the inverse transition temperature of the polymer.

To demonstrate the effectiveness of the composition of the invention, specifically for use as a bio-ink, the following biopolymers were designed **(Table 2),** to subsequently determine which are the best compositions for use as bio-inks.

**Table 2.** Biopolymers, structure and amino acid and nucleotide sequences:

| Name | Structure | Amino acid sequence | Nucleotide sequence |
|---|---|---|---|
| **Biopolymer 1** (93175 Da) | $\{(B_{10}\text{-}C_{60})\}_2\text{-}D$ | SEQ ID NO: 11 | SEQ ID NO: 21 |
| **Biopolymer 2** (101696 Da) | $\{(B_{10}\text{-}C_{60})\text{-}Y\}_2$ | SEQ ID NO: 12 | SEQ ID NO: 22 |
| **Biopolymer 3** (104119 Da) | $\{(B_{10}\text{-}C_{60})\text{-}X\}_2$ | SEQ ID NO: 13 | SEQ ID NO: 23 |
| **Biopolymer 4** (123345 Da) | $\{(B_{10}\text{-}C_{60})\text{-}X\}_2\text{-}D$ | SEQ ID NO: 14 | SEQ ID NO: 24 |
| **Biopolymer 5** (120921 Da) | $\{(B_{10}\text{-}C_{60})\text{-}Y\}_2\text{-}D$ | SEQ ID NO: 15 | SEQ ID NO: 25 |
| **Biopolymer 6** (126393 Da) | $X\text{-}\{(B_{10}\text{-}C_{60})\text{-}Y\}_2\text{-}D$ | SEQ ID NO: 16 | SEQ ID NO: 26 |

Biopolymer 1 (1107 amino acids)

Structure: $(A)\text{-}\{(B_{10}\text{-}C_{60})\}_2\text{-}D$.

Amino acid sequence: SEQ ID NO: 11: MESLLP-$\{[(VPGVG)_2\text{-}(VPGEG)\text{-}(VPGVG)_2]_{10}[VGIPG]_{60}\}_2$-$([VPGIG]_5AVTGRGDSPASS)_6$-V

Coded by nucleotide sequence SEQ ID NO: 21.
The theoretical amino acid composition and the amino acid composition obtained with HPLC with UV (ultraviolet light) detection are presented in Table 3.

**Table 3.** Analysis of the amino acid composition of biopolymer 1.

| | | Glu | Gly | Ile | Leu | Pro | Ser | Val |
|---|---|---|---|---|---|---|---|---|
| Amino acid analysis | Theoretical | 21 | 440 | 120 | 2 | 221 | 1 | 301 |
| | Experimental | 22.01 | 432.83 | 119.34 | 1.91 | 224.02 | 1.09 | 304.80 |

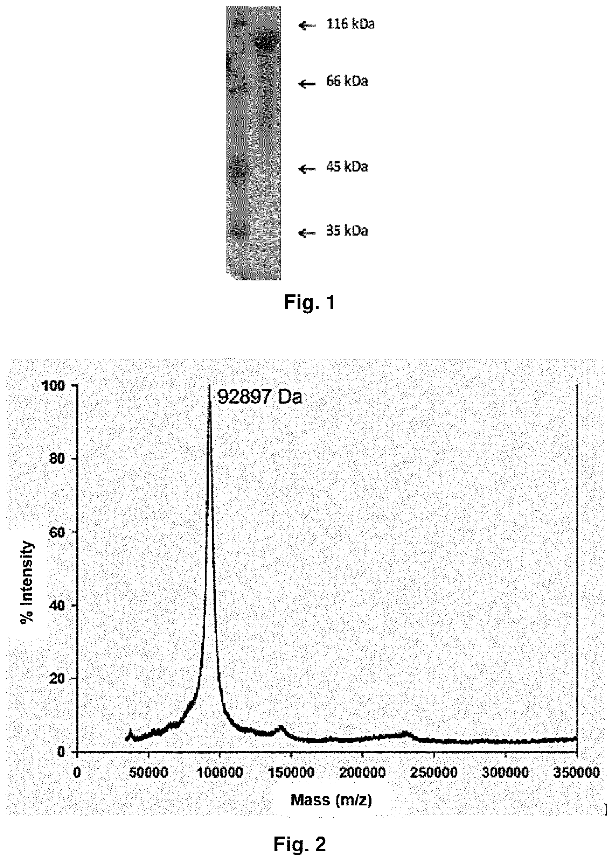

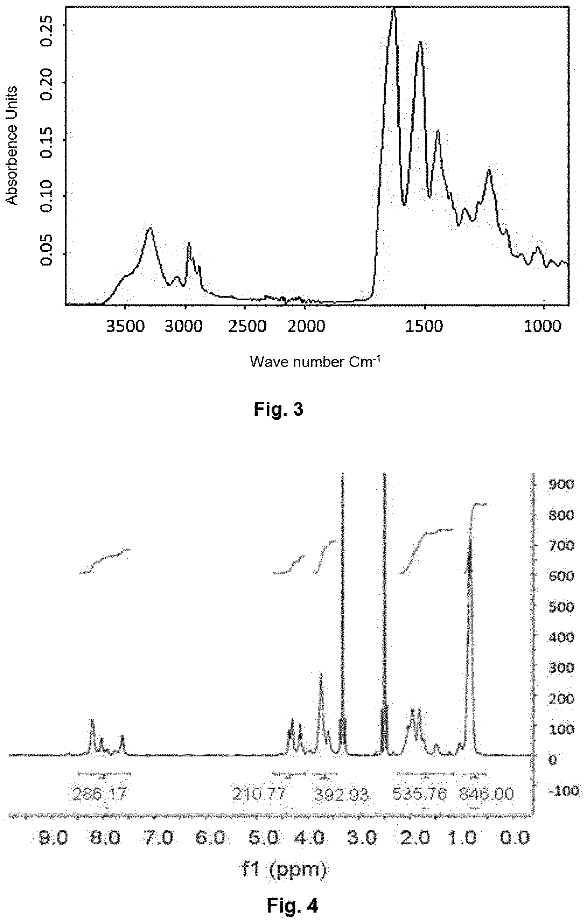

The production yield was 227.65 mg/l.
The theoretical molecular weight for biopolymer 1 is 93175 Da and it was experimentally estimated by polyacrylamide gel electrophoresis **(Figure 1)** and by MALDI-TOF mass spectrometry, resulting in 92897 Da. HPLC, infrared (IR) and nuclear magnetic resonance (NMR) spectra obtained for biopolymer 1 are shown in **Figures 2, 3 and 4,** respectively.
The transition temperature obtained by means of DSC in MQ at pH 7.8 was 19.10 °C, while in 1X PBS at pH 7.65 it was 14.66 °C.

Biopolymer 2 (1233 amino acids)

Structure: (A)-{(B$_{10}$-C$_{60}$)-Y}$_2$

Amino acid sequence SEQ ID NO: 12: MESLLP-{([(VPGVG)$_2$-(VPGEG)-(VPGVG)$_2$]$_{10}$[VGIPG$_{60}$)-[V(GAGAGS)$_5$G]$_2$}$_2$

Coded by nucleotide sequence SEQ ID NO: 22.
The theoretical amino acid composition and the amino acid composition obtained with HPLC with UV (ultraviolet light) detection are presented in Table 4.

**Table 4:** Analysis of the amino acid composition of biopolymer 2.

| | | Ala | Glu | Gly | Ile | Leu |
|---|---|---|---|---|---|---|
| Amino acid analysis | Theoretical | 40 | 21 | 504 | 120 | 2 |
| | Experimental | 33.17 | 24.57 | 501.30 | 122.87 | 2.46 |
| | | **Met** | **Pro** | **Ser** | **Val** | |
| Amino acid analysis | Theoretical | 1 | 221 | 21 | 305 | |
| | Experimental | 1.72 | 232.22 | 17.20 | 294.39 | |

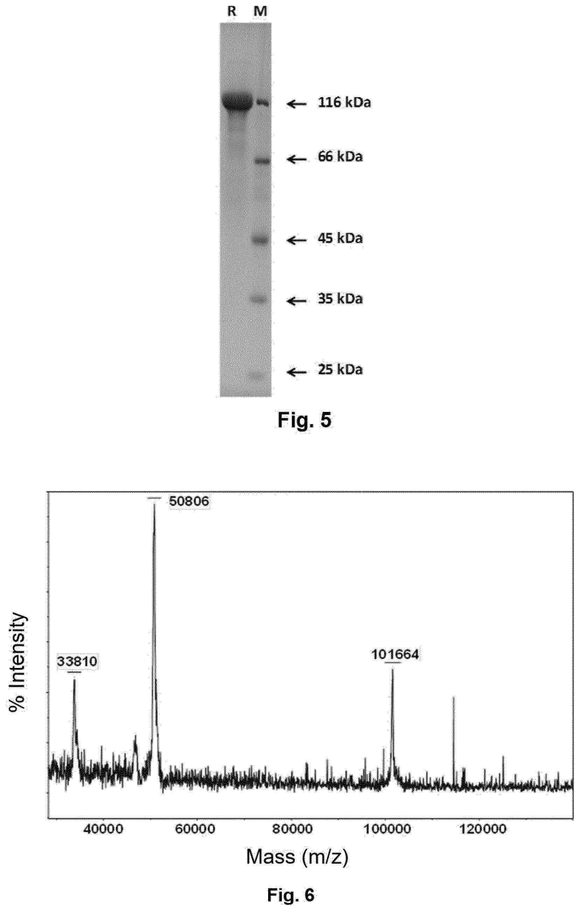

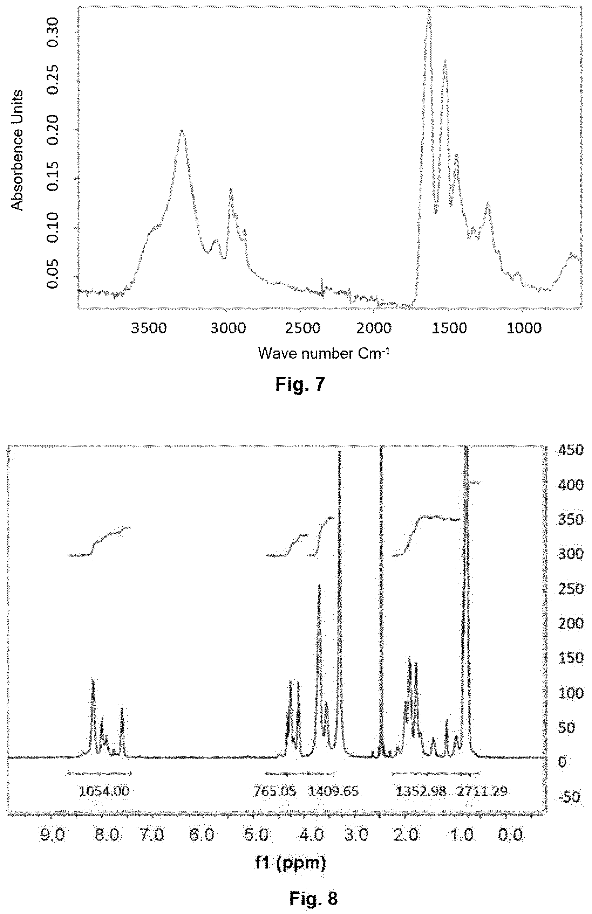

The production yield was 178.9 mg/l.
The theoretical molecular weight for polymer 2 is 101696 Da and it was experimentally estimated by polyacrylamide gel electrophoresis **(Figure 5)** and by MALDI-TOF mass spectrometry **(Figure 6)** resulting in 101664 Da. IR and NMR spectra obtained for biopolymer 2 are shown in **Figures 7 and 8,** respectively.
The transition temperature obtained by means of DSC in MQ at pH 6.14 was 20.08 °C, while in 1X PBS at pH 6.40 it was 16.92 °C.

Biopolymer 3 (1213 amino acids)

Structure: (A)-{(B$_{10}$-C$_{60}$)-X}$_2$

Amino acid sequence SEQ ID NO: 13: MESLLP-{[VPGVG)$_2$-(VPGEG)-(VPGVG)$_2$]$_{10}$[VGIPG]$_{60}$-

[VGGGGGKENQIAIRASFLEKENSALRQEVADLRKELGKCKNILAKYEAGGGG G]}$_2$

Coded by nucleotide sequence SEQ ID NO: 23.
The theoretical amino acid composition and the amino acid composition obtained with HPLC with UV (ultraviolet light) detection are presented in Table 5.

**Table 5:** Analysis of the amino acid composition of biopolymer 3.

| | | Ala | Arg | Asn | Asp | Cys | Glu | Gln | Gly | Ile |
|---|---|---|---|---|---|---|---|---|---|---|
| Amino acid analysis | Theoretical | 12 | 6 | 6 | 2 | 2 | 33 | 4 | 462 | 126 |
| | Experimental | 12.1 7 | 5.35 | 6.17 | 1.94 | 0.97 | 33.53 | 4.51 | 468.61 | 136.08 |

(continued)

|  |  | Leu | Lys | Met | Phe | Pro | Ser | Tyr | Val |  |
|---|---|---|---|---|---|---|---|---|---|---|
| Amino acid analysis | Theoretical | 12 | 12 | 1 | 2 | 221 | 5 | 2 | 305 |  |
|  | Experimental | 11.4 4 | 10.2 0 | 1.72 | 2.70 | 238.81 | 5.68 | 2.72 | 273.06 |  |

The production yield was 517.22 mg/l.

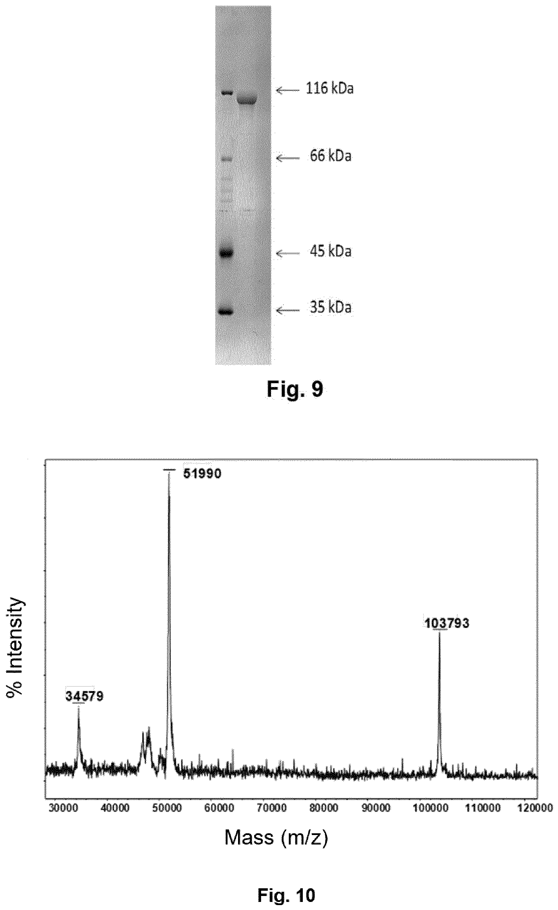

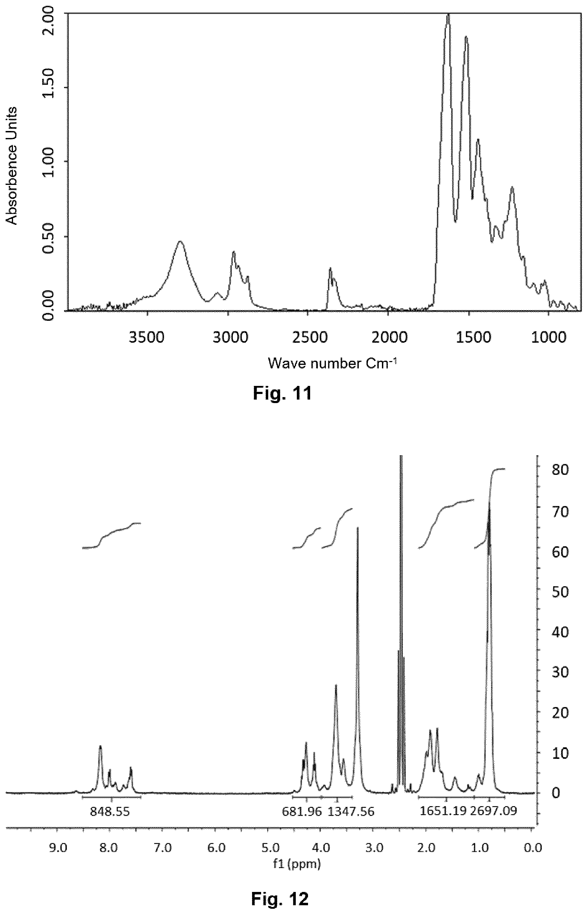

The theoretical molecular weight for polymer F is 104119 Da and it was experimentally estimated by polyacrylamide gel electrophoresis **(Figure 9)** and by MALDI-TOF mass spectrometry **(Figure 10)** resulting in 103,793 Da. IR and NMR spectra obtained for biopolymer 3 are shown in **Figures 11 and 12,** respectively.

The transition temperature obtained by means of DSC in MQ at pH 7.5 was 15.30 °C, while in 1X PBS at pH 7.5 it was 14.18 °C.

Biopolymer 4 (1435 amino acids)

Structure: $(A)-\{(B_{10}-C_{60})-X\}_2-D$

Amino acid sequence SEQ ID NO: 14: MESLLP-{[VPGVG)$_2$-(VPGEG)-(VPGVG)$_2$]$_{10}$[VGIPG]$_{60}$-[VGGGGGKENQIAIRASFLEKENSALRQEVADLRKELGKCKNILAKYEAGGGGG]}$_2$-([VPGIG]$_5$AVTGRGDSPASS)$_6$-V

Coded by nucleotide sequence SEQ ID NO: 24.

The theoretical amino acid composition and the amino acid composition obtained with HPLC with UV (ultraviolet light) detection are presented in Table 6.

**Table 6.** Analysis of the amino acid composition of biopolymer 4.

|  |  | Ala | Arg | Asp+Asn | Cys | Glu+Gln | Gly | Ile |  | Leu |  |
|---|---|---|---|---|---|---|---|---|---|---|---|
| Amino acid analysis | Theoretical | 24 | 12 | 8+6 | 2 | 33+4 | 534 | 156 | 12 |  |  |
|  | Experimental | 18.3 4 | 6.50 | 11.72 | 1.97 | 37.83 | 563.8 | 148.32 | 11.84 |  |  |
|  |  | Lys | Met | Phe | Pro | Ser | Thr | Tyr | Val |  |  |
| Amino acid analysis | Theoretical | 12 | 1 | 2 | 257 | 23 | 6 | 2 | 341 |  |  |
|  | Experimental | 9.75 | 1.39 | 1.16 | 283.18 | 12.65 | 3.83 | 2.67 | 350.61 |  |  |

The production yield was 239.81 mg/l.

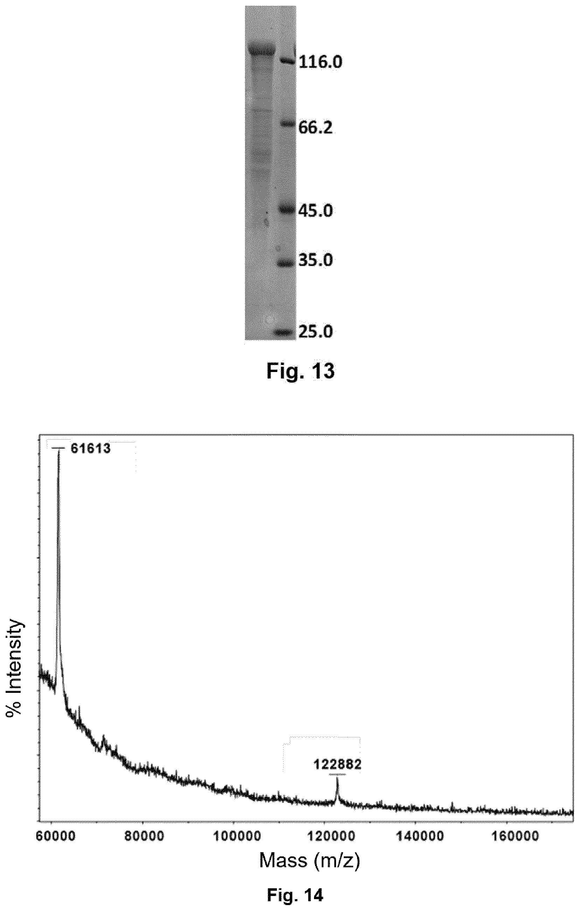

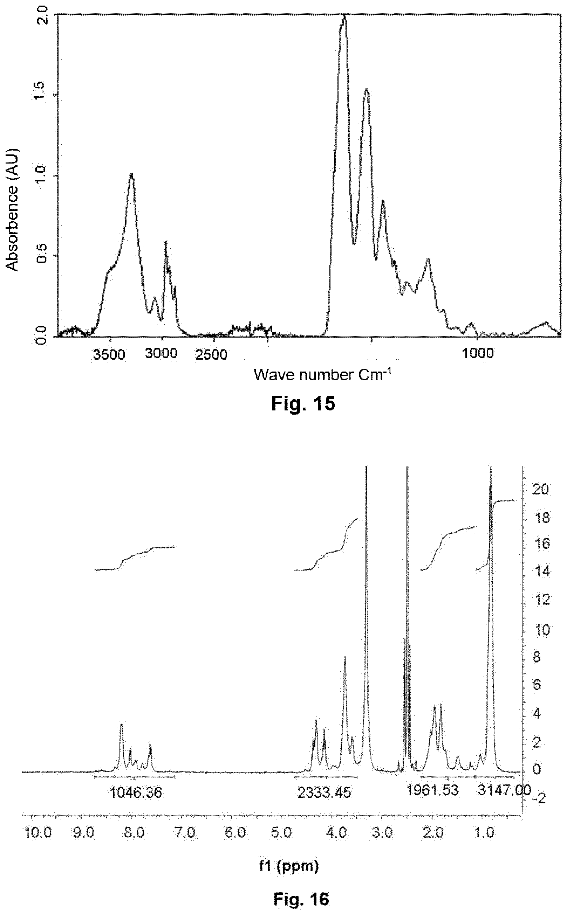

The theoretical molecular weight for biopolymer 4 is 123345 Da and it was experimentally estimated by polyacrylamide gel electrophoresis **(Figure 13)** and by MALDI-TOF mass spectrometry **(Figure 14)** resulting in 122882 Da. IR and NMR spectra obtained for biopolymer 4 are shown in **Figures 15 and 16,** respectively.

The transition temperature obtained by means of DSC in MQ at pH 6.48 was 17.58 °C, while in 1X PBS at pH 6.02 it was 14.92 °C.

Biopolymer 5 (1455 amino acids)

Structure: $(A)-\{(B_{10}-C_{60})-Y\}_2-D$

Amino acid sequence SEQ ID NO: 15: MESLLP-{([(VPGVG)$_2$-(VPGEG)-(VPGVG)$_2$]$_{10}$[VGIPG]$_{60}$)-[V(GAGAGS)$_5$G]$_2$}$_2$-([VPGIG]$_5$AVTGRGDSPASS)$_6$-V

Coded by nucleotide sequence SEQ ID NO: 25.

The theoretical amino acid composition and the amino acid composition obtained with HPLC with UV (ultraviolet light) detection are presented in Table 7.

**Table 7.** Analysis of the amino acid composition of biopolymer 5.

| | | Ala | Arg | Asp | Glu | Gly | Ile |
|---|---|---|---|---|---|---|---|
| Amino acid analysis | Theoretical | 52 | 6 | 6 | 21 | 574 | 150 |
| | Experimental | 50.94 | 4.31 | 5.39 | 29 | 581.86 | 149.41 |
| | | Leu | Met | Pro | Ser | Thr | Val |
| Amino acid analysis | Theoretical | 2 | 1 | 257 | 39 | 6 | 341 |
| | Experimental | 0 | 1.37 | 255.77 | 31.08 | 4.33 | 335.86 |

The production yield was 203.07 mg/l.

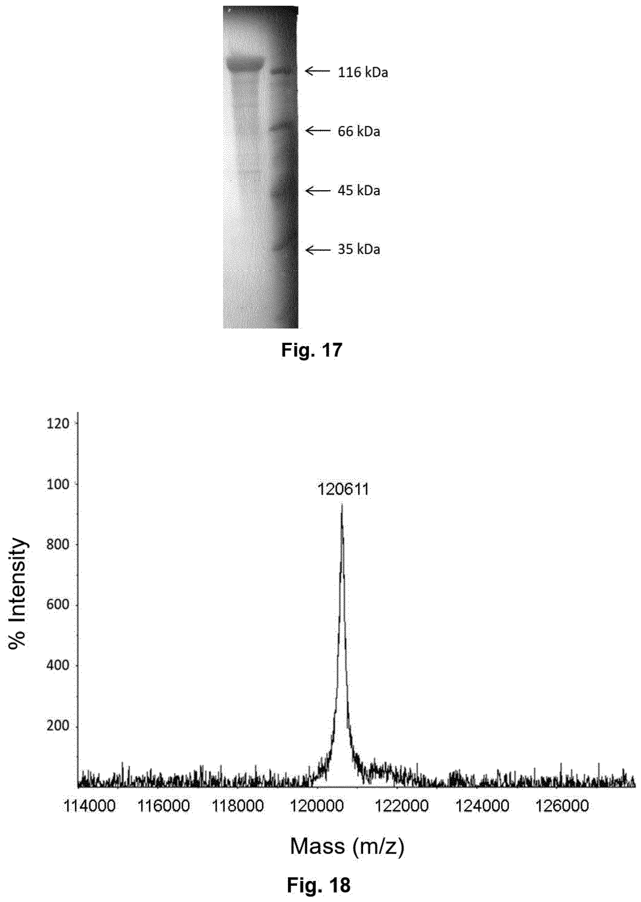

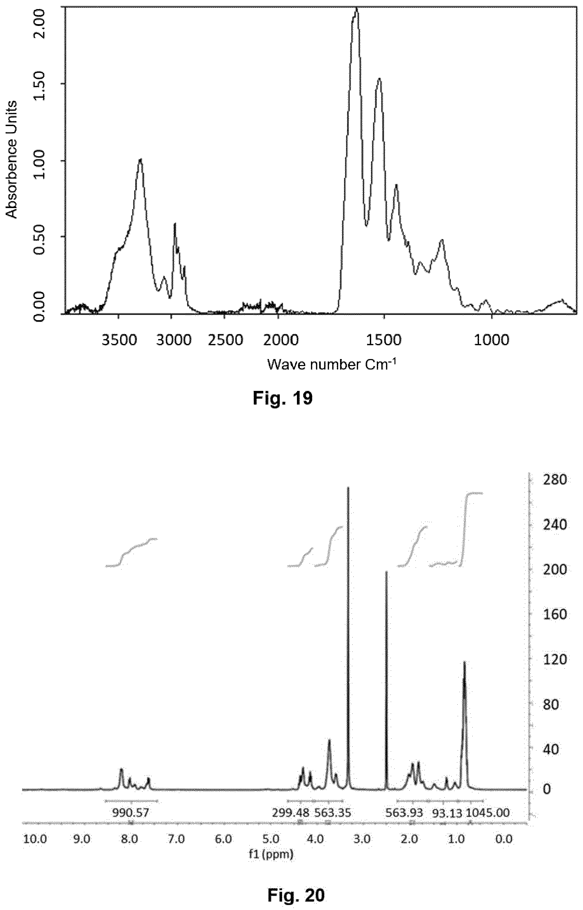

The theoretical molecular weight for biopolymer 3 is 120921 Da and it was experimentally estimated by polyacrylamide gel electrophoresis **(Figure 17)** and by MALDI-TOF mass spectrometry **(Figure 18)** resulting in 120611 Da. IR and NMR spectra obtained for biopolymer 2 are shown in **Figures 19 and 20,** respectively.

The transition temperature obtained by means of DSC in MQ at pH 6.59 was 20.84 °C, while in 1X PBS at pH 7.24 it was 17.26 °C.

Biopolymer 6 (1508 amino acids)

Structure: (A)-X-{(B$_{10}$-C$_{60}$)-Y}$_2$-D

Amino acid sequence SEQ ID NO: 16: MESLLP-[VGGGGGGKENQIAIRASFLEKENSALRQEVADLRKELGKCKNILAKYEAGGGGG]-{([(VPGVG)$_2$-(VPGEG)-(VPGVG)$_2$]$_{10}$[VGIPG]$_{60}$)-[V(GAGAGS)$_5$G]$_2$}$_2$-([VPGIG]$_5$AVTGRGDSPASS)$_6$-V

Coded by nucleotide sequence SEQ ID NO: 26.

The theoretical amino acid composition and the amino acid composition obtained with HPLC with UV (ultraviolet light) detection are presented in Table 8.

**Table 8.** Analysis of the amino acid composition of biopolymer 6.

| | | Ala | Arg | Asn+ Asp | Cys | Glu+Gln | Gly | Ile | Leu |
|---|---|---|---|---|---|---|---|---|---|
| Amino acid analysis | Theoretical | 58 | 9 | 3+7 | 1 | 27+2 | 585 | 153 | 7 |
| | Experimental | 53.78 | 7.72 | 11.15 | 0.32 | 38.85 | 583.93 | 147.01 | 8.90 |
| | | Lys | Met | Phe | Pro | Ser | Thr | Tyr | Val |
| Amino acid analysis | Theoretical | 6 | 1 | 1 | 257 | 41 | 6 | 6 | 343 |
| | Experimental | 12.37 | 0.98 | 1.13 | 260.95 | 35.50 | 4.54 | 13.91 | 323.98 |

The production yield was 116 mg/l.

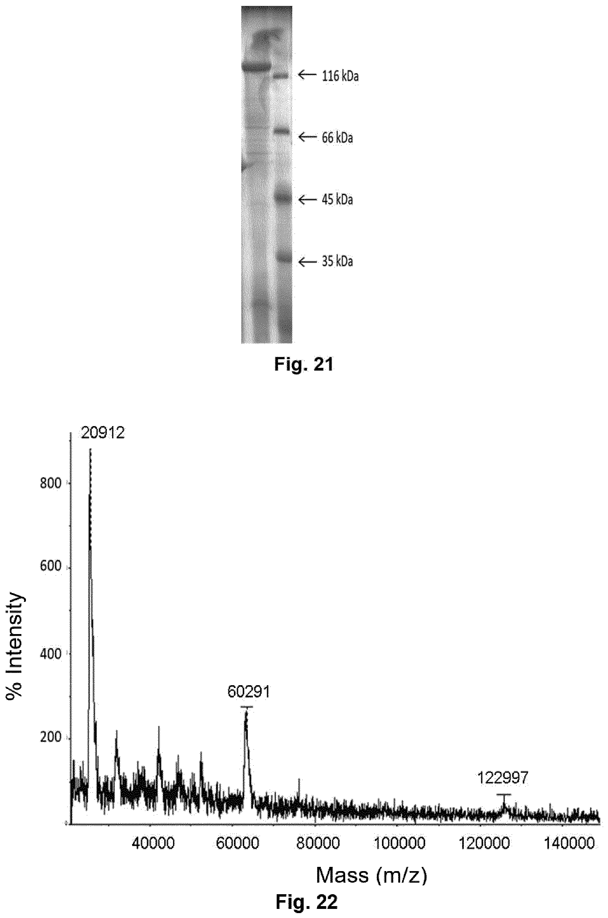

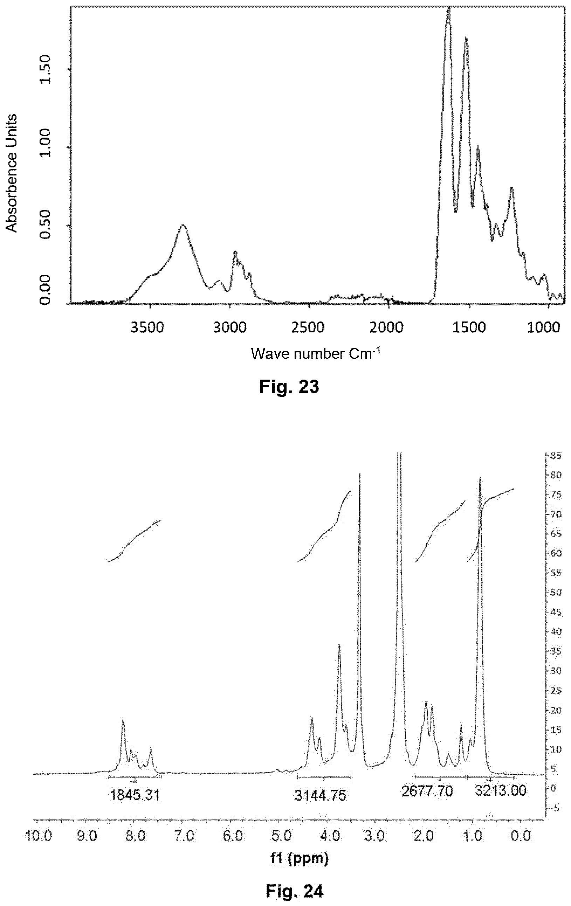

The theoretical molecular weight for biopolymer 6 is 126393 Da and it was experimentally estimated by polyacrylamide gel electrophoresis **(Figure 21)** and by MALDI-TOF mass spectrometry **(Figure 22)** resulting in 125857 Da. IR and NMR spectra obtained for biopolymer 6 are shown in **Figures 23 and 24,** respectively.

The transition temperature obtained by means of DSC in MQ at pH 7.50 was 20.42 °C, while in 1X PBS at pH 7.50 it was 17.41 °C.

**Example 2. Determination of the composition of the bio-ink of the invention which allows optimal printing.**

3D printing with the different biopolymers described in Table 2, or with mixtures thereof, are performed taking into account the inverse transition temperature of each of them. Said transition temperature together with the specific properties of the compositions of the biopolymers of the invention cause the potential bio-inks to gel by means of a simple change in temperature.

The experimental system used comprises a REGEMAT 3D printer on which there has been installed a head connected to a cooling bath, which allows the injection temperature to be kept at 4 °C. Moreover, the printer has a heating bed which is kept at 30 °C during the printing process.

In the case of biopolymer 1 (SEQ ID NO: 11), gelling is due to the hydrophobic intermolecular forces present between its blocks C (hydrophobic) and B (hydrophilic). Block D, specifically comprising the peptide RGD additionally introduced in its sequence, does not affect gel formation, but rather is introduced to provide the biopolymer with biofunctionality. This biopolymer 1 will be used as a negative control of bioprinting, given that it does not contain any of monomer X, monomer Y or both.

The rest of biopolymers 2 (SEQ ID NO: 12), 3 (SEQ ID NO: 13), 4 (SEQ ID NO: 14), 5 (SEQ ID NO: 15) and 6 (SEQ ID NO: 16), containing base monomers C (hydrophobic) and B (hydrophilic), in addition to other monomers X and/or Y, also show these hydrophobic interactions. They all further contain block D comprising, in the examples shown, specifically peptide RGD to provide the biopolymer with biofunctionality, allowing cell adhesion to be induced.

Biopolymers 4 (SEQ ID NO: 14) and 3 (SEQ ID NO: 13) comprise the *zipper* sequence (SEQ ID NO: 4), which allows the formation of alpha helices through the interaction of electrostatic forces between charged amino acids, contributing to the stability of the polymer. Biopolymers 5 (SEQ ID NO: 15) and 2 (SEQ ID NO: 12) show the same hydrophobic interactions, but in this case stabilized as a result of the formation of beta sheets originating from the silk sequence (SEQ ID NO: 8), by means of the formation of hydrogen bonds between amido and carboxyl groups present in their amino acids.

For the particular case of biopolymer 5 (SEQ ID NO: 15), a comparison between its gelling when said biopolymer has been subjected to a pre-curing treatment (referred to hereinafter as pre-cured biopolymer 5) or when it has not been subjected to said treatment (which will continue to be called biopolymer 5) has been carried out. The pre-curing treatment is performed due to the variability of biopolymer 5 in terms of its structure at a molecular level, where it may present a different degree of formation of beta sheets which affect its mechanical characteristics. During the production and purification of biopolymer 5, the formation of beta sheets occurs through hydrogen bonds. Said cross-linking is not homogeneous among the different batches of biopolymer, generating batches having a different initial cross-linking. Breaking the hydrogen bonds with the pre-curing treatment ensures that the initial state of beta sheet formation is the same for all the batches. To carry out the pre-curing treatment, first the biopolymer is homogenized by breaking its intermolecular forces using formic acid, which allows starting from a state with an absence of beta sheets. After this state, the biopolymer is subjected to curing at 37 °C for 24 hours, favoring the formation of beta sheets. The same initial state is thereby ensured in all the batches of this polymer and the process starts from a pre-gelled state that is foreseeably more suitable for printing.

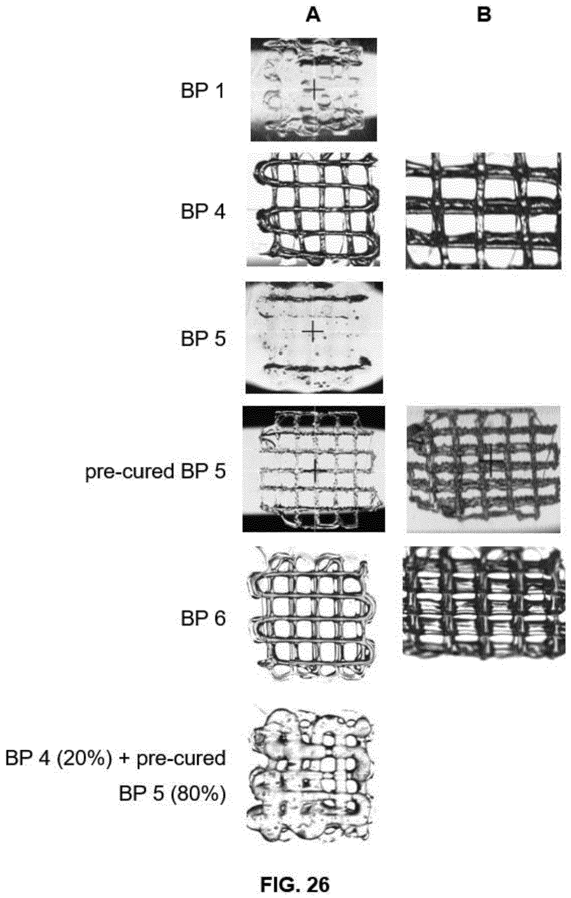

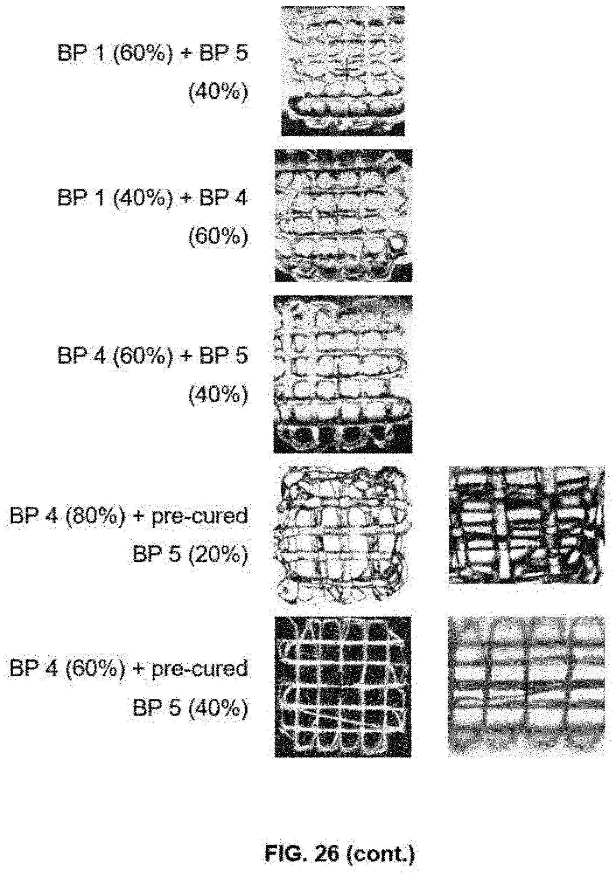

Both biopolymer 1 (SEQ ID NO: 11) mixed together with biopolymer 4 (SEQ ID NO: 14), and biopolymer 1 (SEQ ID NO: 11) mixed with pre-cured biopolymer 5 (SEQ ID NO: 15) also serve as a negative control for printing. Their printing demonstrates that optimal printing is obtained only if the mixture comprises both reinforcement sequences **(Figure 26).**

Moreover, for the purpose of confirming whether the mixtures of polymer 4 and pre-cured polymer 5 behave the same way when monomers X and Y are located in the same biopolymer, biopolymer 6 (SEQ ID NO: 6) was synthesized. This biopolymer 6 presents both the initial hydrophobic interactions and the electrostatic and hydrogen bond interactions originating from the *Zipper* and Silk sequences.

Different printing operations were carried out with compositions comprising biopolymers 1, 4, 5, pre-cured biopolymer 5 and biopolymer 6, alone or combined. To this end, by means of the REGEMAT 3D printer software, a grating is designed, measuring 10x10 mm, with a height of 1.30 mm (corresponding to 6 layers of height), and a porosity of 1.5 mm arranged at an angle of 90°. The different compositions of the biopolymers of the invention will be injected with a 0.25 mm nozzle and 0.06 mm/s flow. The flow is adapted if needed in each case to enable making lines of similar width which allow the structures to be better compared to one another.

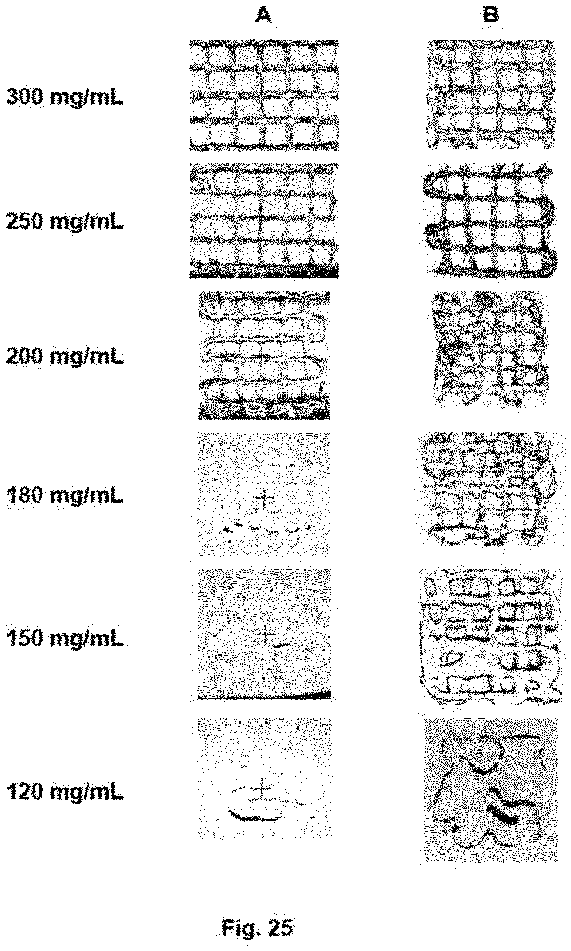

The optimal printing concentration of pre-cured biopolymer 5 dissolved at different concentrations of 1x PBS (120, 150, 180, 200, 250 and 300 mg/ml) is estimated to be 250 mg/ml, as observed in **Figure 25,** given that said concentration is the concentration at which the viscosity of the solution is suitable for the "injection" process by means of the 3D printer. Therefore, the concentration of 250 mg/ml is selected for the comparison of the various printing operations. For the case of biopolymer 4, as also observed in **Figure 25,** the suitable concentration for the injection process in the 3D printer is 250 mg/ml.

Printing the designed gratings allows semi-quantification of the printability of the various biopolymers of the invention to be performed by measuring printability, which gives an idea as to the similarity the printed structure has with respect to designed structure. In this case, square pores are designed and printed, therefore a parameter (Pr) is established for measuring the similarity of the printing operations with respect to these squares.

The bio-ink demonstrating good printability must be deposited through the extrusion of filaments having a constant morphology which allow being deposited in height, without being fused to one another. If the bio-ink demonstrates less printability, this does not occur and the filaments tend to collapse and fuse to one another, forming porosities that tend to be circular. Circularity is therefore defined as:

$$C = \frac{4\pi A}{L^2}$$

Wherein L defines the perimeter and A the area. The closer the obtained value is to 1, the greater the circularity will be, with 1 being a perfect circle.

In the case of square shapes, circularity will be $\pi/4$, and parameter Pr based on the square shape can be defined as:

$$Pr = \frac{\pi}{4} \cdot \frac{1}{C} = \frac{L^2}{16A}$$

For ideal printability, the interconnected pores will be square and the Pr value will be 1.

The determination of parameter Pr is performed by taking photographs with a LEICA DMS 1000 digital microscope, and the optical images taken are analyzed through Image J software (n=5) **(Figure 26).**

The results of said parameter for the assayed samples are summarized in **Table 9.**

Table 9: Measurement of printability (Pr) obtained in each bio-ink printed at a final concentration of 250 mg/ml.

| BIO-INKS (250 mg/ml) | Pr VALUE |
|---|---|
| biopolymer 1 | - |
| biopolymer 4 | 0.94 ± 0.03 |
| biopolymer 5 | - |
| pre-cured biopolymer 5 | 0.90 ± 0.09 |
| biopolymer 6 | 0.97 ± 0.10 |
| biopolymer 1 (40 %) + biopolymer 4 (60 %) | 0.77 ± 0.04 |
| biopolymer 1 (60 %) + biopolymer 5 (40 %) | 0.73 ± 0.10 |
| biopolymer 4 (60 %) + biopolymer 5 (40 %) | 0.86 ± 0.04 |
| biopolymer 4 (20 %) + pre-cured biopolymer 5 (80 %) | 0.65 ± 0.32 |
| biopolymer 4 (60 %) + pre-cured biopolymer 5 (40 %) | 0.96 ± 0.04 |
| biopolymer 4 (80 %) - pre-cured biopolymer 5 (20 %) | 0.93 ± 0.02 |

Another parameter, in this case qualitative, for determining printability of bio-inks is fibrillar observation, in other words, if the deposition of the polymers is deposited layer-by-layer and the deposited fibers are observed, the structure will tend to collapse less than if these fibers fuse to one another, causing lower shape fidelity.

In **Figure 26,** the photographs of the printing that was performed are observed. After measuring parameter Pr, it is observed that after a Pr value of 0.90, the printing operations are performed in a controlled manner, allowing the deposition of fibers which do not mix with one another when they are deposited, maintaining their fibrillar structure in height.

As observed in **Figure 26,** biopolymer 1 does not gel after printing, therefore it does not maintain its structure. This behavior corresponds with what is expected, given that this polymer does not contain monomers X and/or Y, which allow for stability of the gel formed.

Unlike what occurs with biopolymer 1, when biopolymers have monomers X and/or Y, as in the case of biopolymers 4, 5 and pre-cured biopolymer 5, printing is performed with same. The biopolymer 5 allows for printing thereof, but the printed structure rapidly disaggregates such that it does not allow its printability to be measured. In contrast, pre-cured polymer 5 shows over-gelling, which is observed in the non-linear deposition of the fibers, despite its high printing fidelity. Therefore, the fact that the sample gels due to a change in temperature is not a sufficient condition as would have been expected for achieving a good bio-ink for this system, and thus obtaining printed surfaces showing a structure which truly resembles the designed structure. The phenomenon that accompanies gelling and the modifications in the mechanical properties of the material before and after gelling are determining factors and may be inadequate, without being able to predict which material is suitable as a bio-ink.

Lastly, biopolymer 4 shows reliable structures with Pr values of 0.94 **(Figure 26)**.

Given that pre-cured polymer 5 shows a higher printability parameter than polymer 5, for demonstrating the synergistic effect of monomers X and Y, pre-cured polymer 5 is selected as a carrier of monomer Y, such that the following mixtures are printed: biopolymer 4 (80 % by weight) + pre-cured biopolymer 5 (20 % by weight); biopolymer 4 (60 % by weight) + pre-cured biopolymer 5 (40 % by weight) and biopolymer 4 (20 % by weight) + pre-cured biopolymer 5 (80 % by weight). The printing of the proportions of biopolymer 4 (60 % by weight) + pre-cured biopolymer 5 (40 % by weight) show the highest Pr value obtained in the printed mixtures, therefore this mixture can be considered optimal for 3D printing. The lowest value is obtained when the proportion of pre-cured biopolymer 5 is higher. Furthermore, printing of the mixture of biopolymer 4 (60 % by weight) + biopolymer 5 (40 % by weight) allows observing that when the printing mixture for polymers of this type is optimal, if biopolymer 5 is not subjected to pre-curing, printability decreases, making it necessary to use pre-cured biopolymer 5 if better structure fidelity is to be obtained.

To confirm the synergy between monomers X and Y, the mixtures comprising biopolymer 5 (40 % by weight) + biopolymer 1 (60 % by weight) and the mixture of biopolymer 4 (60 % by weight) + biopolymer 1 (40 % by weight) are also printed. Said mixtures show printing with worse printability, confirming that to obtain good printability, the mixture of biopolymers comprising monomers X and Y is necessary.

Lastly, the printing of biopolymer 6 shows very good shape fidelity in which the deposition of fibers is observed, such that they do not end up being completely superimposed. The highest printability value, corresponding to 0.97, is obtained for this polymer. Although the Pr value obtained for this polymer is not very different from that of the mixture comprising biopolymer 4 (60 % by weight) + pre-cured biopolymer 5 (40 % by weight), greater deposition ease is observed in printing, with better control over the deposited fibers.

**Example 3. Mechanical properties of the compositions of the invention.**

A rheological analysis was carried out to analyze the mechanical properties of the compositions of the invention for use as bio-inks. Those compositions which demonstrated having greater printability are selected, corresponding with the compositions comprising biopolymer 4, biopolymers 6, pre-cured biopolymer 5 and the mixture of biopolymers 4 (60 % by weight) + pre-cured biopolymer 5 (40 % by weight).

The rheological study is carried out with a TA Instruments AR 200 EX rheometer, equipped with a peltier plate, and a geometry of 40 mm in diameter. All the analyzed compositions are kept at 4 °C during the assays.

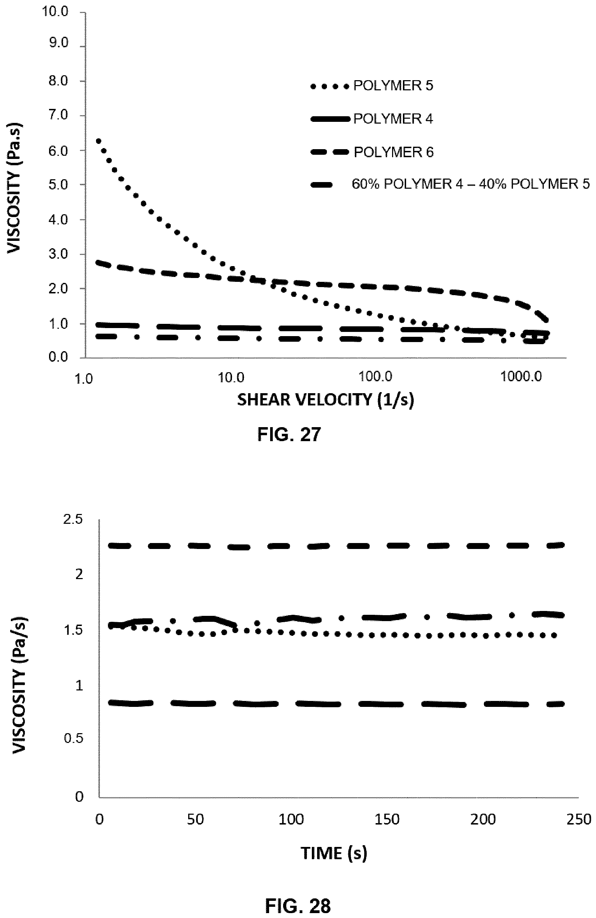

The first characterization is based on the study of the variation in viscosity experienced by the compositions of the invention for use as bio-inks when subjected to an increasing shear velocity **(Figure 27)**. Both biopolymer 4 and the mixture comprising biopolymer 4 (60 % by weight) + pre-cured biopolymer 5 (40 % by weight) do not show a decrease in viscosity when shear velocity is increased, therefore behaving like Newtonian fluids showing relatively low viscosities (about 1 Pa.s). The fact that they behave like low-viscosity Newtonian fluids allows controlled depositions to be performed with lower shear stresses, thus protecting the cells which are embedded in said compositions. Moreover, the composition formed by pre-cured biopolymer 5 presents a decrease in viscosity when shear stress is increased, starting from viscosities of 10 Pa.s and stabilizing at viscosities of 1 Pa.s when the bio-ink is subjected to high shear velocities. This behavior of the bio-ink is similar to that described by a pseudoplastic fluid.

Furthermore, the composition comprising biopolymer 6 has a viscoplastic (or Bingham plastic) behavior, showing a viscosity of 2.35 Pa.s until reaching a critical deformation stress corresponding to 248.6 1/s, after which viscosity starts to slightly decrease until reaching 1.41 Pa.s.

It can be concluded that the compositions comprising monomer Y show a pseudoplastic behavior in the bio-inks. In the case of biopolymer 6, This behavior experiences a delay, which can start to be observed once a critical deformation stress has been exceeded. Said behavior corresponds to a plastic showing a viscoplastic or Bingham plastic behavior.

To simulate the injection process to which the compositions of the invention are subjected when they are used as bio-inks in a printer, a thixotropic analysis thereof was carried out. This analysis consists of subjecting the biopolymers to a high shear velocity for a short period of time, trying to mirror the forces to which the bio-inks are subjected when going through a needle having a very small diameter in the printing process.

The results obtained in the thixotropic analysis of the compositions described above clearly showed that there is no variation in their viscosity when the injection process is simulated, in other words, none of the analyzed compositions varied in viscosity when subjected to high shear stress for a short period of time **(Figure 28).**

The variation in viscosity when an increase in temperature occurs was then analyzed. The analysis determines the ideal temperature at which the print bed should be pre-heated to achieve a higher viscosity in the bio-ink, and, therefore, a higher printing resolution.

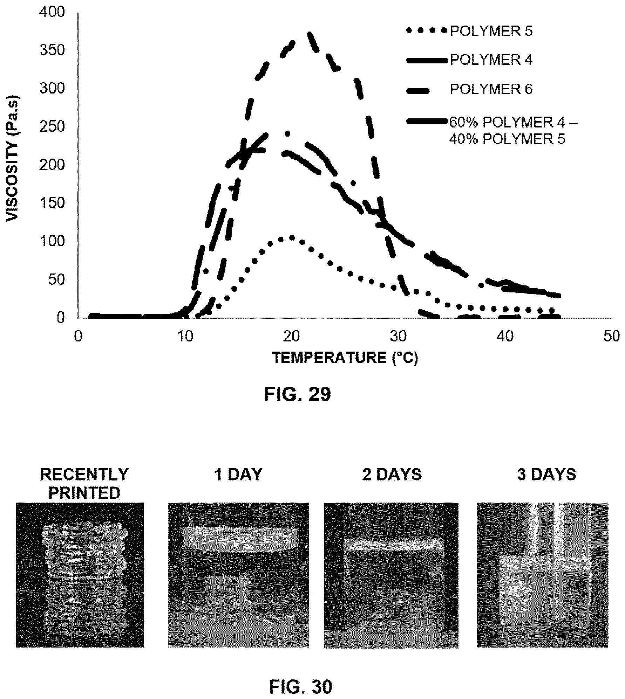

The results demonstrate that the polymer viscosity of the analyzed compositions increases as the temperature increases until reaching a maximum value that depends on each composition, and it ranges between 18-22 °C, and subsequently said viscosity gradually decreases as the temperature continues to increase **(Figure 29).** This maximum viscosity reached is different for each bio-ink composition and said maximum is reached at a different temperature. Thus, as observed in **Figure 29,** the composition comprising biopolymer 4 has a viscosity of 212.5 Pa.s at a temperature of 15.0 °C; the composition comprising pre-cured biopolymer 5 has a viscosity of 90.9 Pa.s at a temperature of 18.4 °C; the composition comprising biopolymer 6 has a viscosity of 371 Pa.s at a temperature of 21.7°C; and the composition comprising the mixture of biopolymer 4 (60 % by weight) + pre-cured biopolymer 5 (40 % by weight) has a viscosity of 242.6 Pa.s at a temperature of 19.5 °C.

Taking said results into account, there is a correlation between the maximum viscosities achieved by the studied bio-inks and their printability. Thus, bio-inks which show lower printability (bio-inks comprising biopolymers 4 and pre-cured biopolymer 5, respectively) reach a lower maximum viscosity than the biopolymers having a higher printability (bio-inks comprising the mixture of biopolymer 4 (60 % by weight) + pre-cured biopolymer 5 (40 % by weight) and the bio-ink comprising biopolymer 6). This is explained taking into account that the higher the viscosity a bio-ink demonstrates to present, the greater its printing fidelity will be.

Likewise, it has been observed that in all the bio-inks there is a critical temperature point after which viscosity starts to gradually increase, and said critical temperature point corresponds to about 8.5 °C for bio-inks comprising biopolymer 4 and the mixture of biopolymer 4 (60 % by weight) + pre-cured biopolymer 5 (40 % by weight), and 11 °C for bio-inks comprising pre-cured biopolymer 5 or biopolymer 6. This critical point indicates that in order to print with low viscosities, the bio-inks must be kept below this temperature in the reservoir.

## EXAMPLE 4. Fluid stability of bio-inks.

Next the stability of the compositions described in the present invention in a selected fluid was analyzed, said fluid being 1x PBS for the case of the present example. Given that the structures printed with the compositions described in the present invention will be used for *in vitro* cultures, or for tissue models, the structures must be able to remain stable in aqueous media for prolonged periods of time. For this example, cylinders are designed measuring 6 mm in diameter and 1.5 cm in height, and printing operations were carried out through a nozzle measuring 0.25 mm in diameter. The assayed compositions were the compositions comprising biopolymer 4, pre-cured biopolymer 5, biopolymer 6 or the mixture of biopolymer 4 (60 % by weight) + pre-cured biopolymer 5 (40 % by weight).

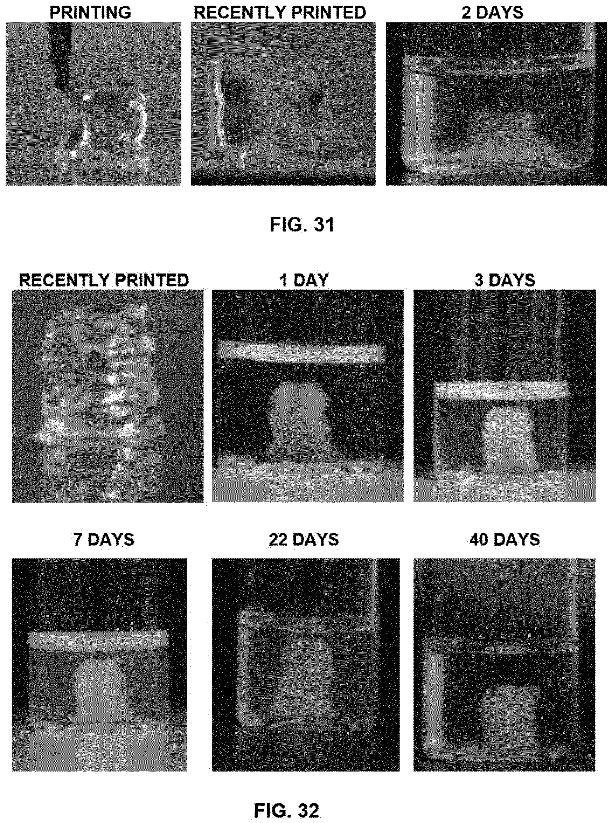

Printing with the composition comprising biopolymer 4 at a concentration of 250 mg/ml in 1x PBS shows good structural maintenance, but it is not capable of being maintained over time **(Figure 30).** On the contrary, printing operations with the compositions comprising pre-cured biopolymer 5, under the same conditions as those mentioned above, show lower shape fidelity and collapsing thereof in a short time interval, which does not allow structures to be printed which suitably maintain their shape in height, despite showing stability over time **(Figure 31).**

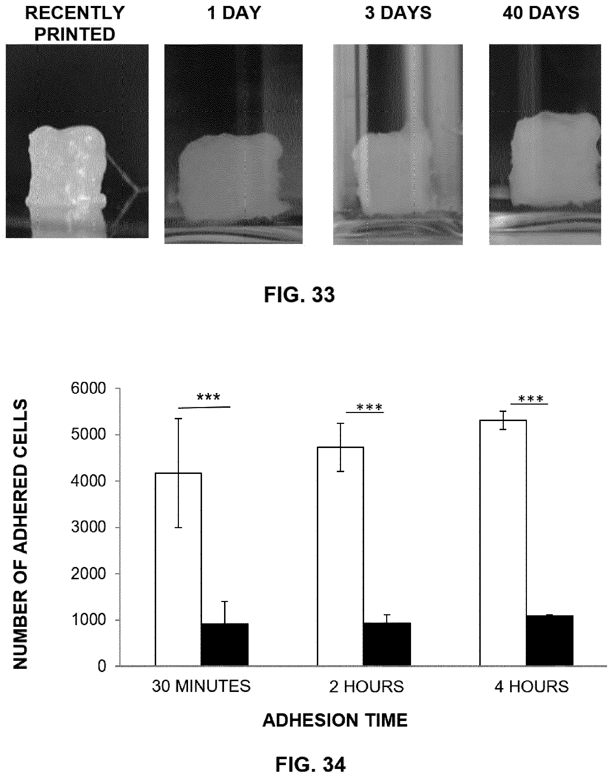

On the contrary, printing with the composition comprising the mixture of biopolymer 4 (60 % by weight) + pre-cured biopolymer 5 (40 % by weight) solves the drawbacks that said compositions present separately when used as bio-inks. This mixture allows printing showing good shape fidelity and structural maintenance over time to be performed, which allows complex structures to be made **(Figure 32).** Likewise, the bio-ink comprising the composition with biopolymer 6 also shows structural maintenance over time, since it comprises monomers X and Y in its sequence **(Figure 33).**

## EXAMPLE 5. Evaluation of cell viability and cytotoxicity of the compositions.

Once the printability of the compositions of the invention as bio-inks and their consistency over time were evaluated, their cytotoxicity and cell viability were evaluated using human fibroblast cell line HFF-1. To this end, porous circular gratings are printed measuring 5 mm in diameter and 1 mm in height, with a square porosity of 1 mm sidewise.

The composition selected for performing the assays was the composition comprising the mixture of biopolymer 4 (60 % by weight) + pre-cured biopolymer 5 (40 % by weight), since, as demonstrated in the previous examples, these are the compositions which have demonstrated the best printability and stability over time, since they comprise monomers X and Y. To determine the bioactivity that said composition presents when it comprises in the sequence of biopolymers comprising monomer D, specifically monomer D comprising the RGD sequence, more specifically monomer D comprising the sequence SEQ ID NO: 6, a composition comprising the mixture of biopolymer 3 (60 % by weight) + biopolymer 2 (40 % by weight) is used as a negative control, wherein said composition comprises the same structure as the other assayed composition but lacks monomer D, which allows the functionalization and therefore the bioactivity of the compositions.

On each surface printed with each of the aforementioned compositions, 10,000 cells will be seeded. An Alamar blue assay is performed to study early cell viability and proliferation. Alamar Blue is a reagent containing a fluorescent indicator which is reduced by changing color as a result of cellular metabolic activity, allowing the quantitative determination of cell viability and cytotoxicity. Through the Alamar blue assay, early cell viability is observed at 30 minutes, 2 hours and 4 hours after seeding with each of the assayed compositions. The number of cells adhered on the surfaces is calculated by means of a calibration line, and it is clearly shown that the number of cells which adhere is significantly higher in those gratings which have been printed with the composition comprising the mixture of biopolymer 4 (60 % by weight) + pre-cured biopolymer 5 (40 % by weight) comprising monomer D, with respect to the composition comprising the mixture of biopolymer 3 (60 % by weight) + biopolymer 2 (40 % by weight), which does not comprise monomer D **(Figure 34)**. Therefore, it can be deduced that the presence of the RGD integrin adhesion sequence in bio-inks allows for and improves early cell adhesion.

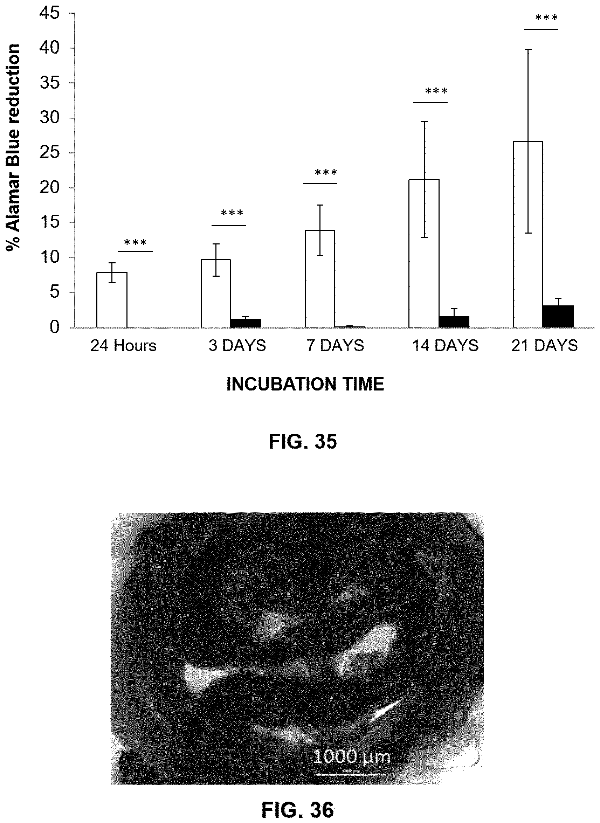

Furthermore, fibroblast cell proliferation on the gratings printed with the aforementioned compositions for long periods of time, that is, 21 days, is also analyzed. The results show a gradual increase in the percentage of reduction of AlamarBlue in both compositions over time, starting from a percentage of 4.4 % in the beginning up to 64.1 % in the case of the composition comprising the mixture of biopolymers 4 (60 % by weight) + pre-cured biopolymers 5 (40 % by weight), and from 2.2 % in the beginning up to 53.2 % in the case of the composition comprising the mixture of biopolymers 3 + (60 % by weight) + biopolymer 2 (40 % by weight) **(Figure 35)**. Said percentage is significantly higher in the case of gratings which have been printed with the composition comprising the mixture with monomer D in its sequence.

To demonstrate that the cells have adhered to the gratings printed with the aforementioned compositions, DAPI/Phalloidin staining was carried out. DAPI staining is used to stain the adenine-thymine bonds of the DNA present in the cell nucleus, while Phalloidin is used for staining actin filaments, allowing the observation of the rest of the cytoplasm. The combination of both staining techniques allows cell morphology to be observed.

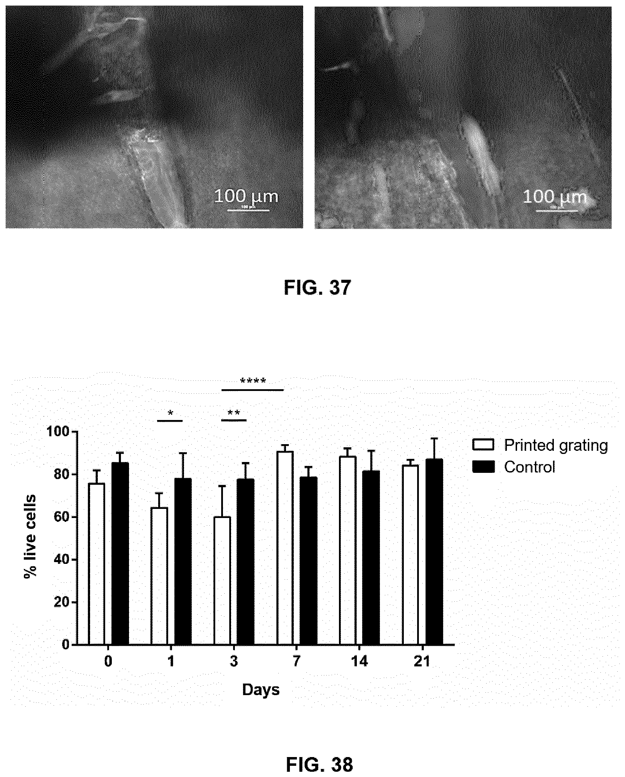

DAPI/Phalloidin staining is performed 14 days after of culturing fibroblasts with gratings printed with the composition comprising biopolymer 4 (60 % by weight) + pre-cured biopolymer 5 (40 % by weight). In **Figure 36,** it can be observed how the cells have adhered to the printed gratings, preferably being situated longitudinally, forming a three-dimensional matrix. The cells are also observed arranged at different heights corresponding to the deposition of the different fibers **(Figure 37),** demonstrating that the morphology or structure of the grating conditions cell arrangement and growth.

**EXAMPLE 6. Evaluation of cell viability and cytotoxicity of cells embedded in the bio-ink (biopolymer 6) prior to bioprinting.**

Next, the cell viability and morphology shown by human fibroblasts HFF-1 when printed together with biopolymer 6 are evaluated.

To this end, HFF-1 cells ($6\times10^6$ cells/ml) are mixed together with the biopolymer 6 dissolved in DMEM and printed in porous circular gratings measuring 5 mm in diameter and 1 mm in height, with a square porosity of 1 mm sidewise. Once the surfaces have been printed under sterility, they are immersed in the cellular medium and incubated for 21 days.

First, a LIVE/DEAD™ staining is performed on the printed surfaces. Staining of this type serves to determine cell viability by means of staining live and dead cells. By obtaining photographs of different fields of the printed grating, cells can be counted and a percentage of viability (percentage of live cells) established. In order to know if cell viability is modified due to the printing process, control is performed, said control being a deposition of the same biopolymer and the same cellular concentration mixed and deposited on a grating without having been subjected to the 3D bioprinting process.

The analysis of the presence of live and dead cells is performed 4 hours after printing, and on different days: day 1, day 3, day 7, day 14 and day 21. In **Figure 38,** a viability of 76 % is observed in cells printed together with biopolymer 6 4 hours after being printed. However, viability values increased significantly after 7 days of culture, reaching 90 % cell viability. During the first few days of cell culture, a noticeable difference is observed between the cell viability obtained in the printed gratings and their corresponding control. These results suggest that during the first few days of culture, the extrusion of biopolymer 6 negatively affects cell viability, although said long-term cell viability is not affected.

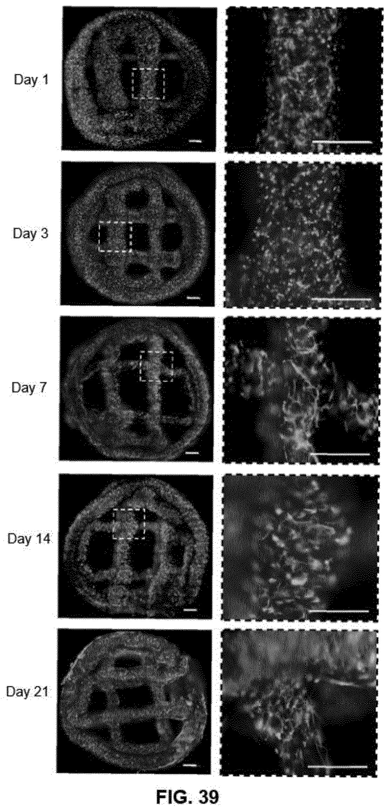

Cell morphology, reorganization and proliferation of HFF-1 were studied by means of light microscopy through DAPI/Phalloidin staining, explained above. This staining was performed after printing biopolymer 6 mixed together with cells ($6 \times 10^6$ cells/ml) after 1, 3, 7, 14 and 21 days. As observed in **Figure 39,** from the first day, cells are homogeneously distributed on the printed surfaces, which denotes a good distribution of nutrients through the gratings which keeps the cells located on both the inner and outer parts thereof. In addition, during the first steps/days of culture, the cells remain round, but after the three first days of incubation, they start to develop an elongated and fibrous shape, which is characteristic of this cell type (fibroblasts), being completely spread out 7 days after culture. In this step, the cells started to form aggregates along the structures and maintained growth and proliferation for the remaining days of culture, up to 21 days, when the experiment ended.

Therefore, as shown in the examples included herein, the compositions comprising the mixture of biopolymer 4 + pre-cured biopolymer 5, as well as the compositions comprising biopolymer 6, can be used as bio-inks. Said compositions present good printability, allowing structures that are resolute in height and stable over time to be printed **(Table 9, Figures 30 and 31),** specifically as a result of the presence in the sequences thereof of monomers X and/or Y. In addition, they present low viscosities which facilitate printing at low temperatures **(Figure 27),** and a rapid increase in viscosity when temperature increases **(Figure 29),** which facilitates the printed structure remaining stable in the printing process. Additionally, they allow cell adhesion and proliferation as a result of the presence of monomer D.

**Claims**

1. A composition comprising a biopolymer comprising monomers B, C, X and Y, or at least two biopolymers comprising monomers B, C and X and monomers B, C and Y, respectively, wherein

   B comprises repeats of pentapeptide VPGXG (SEQ ID NO: 7), preferably B is an amino acid sequence comprising SEQ ID NO: 2,
   C is an amino acid sequence comprising SEQ ID NO: 3,
   X is an amino acid sequence comprising SEQ ID NO: 4, and
   Y comprises 1 to 15 repeats of SEQ ID NO: 8, preferably Y is an amino acid sequence comprising SEQ ID NO: 5.

2. The composition according to claim 1, wherein the biopolymer further comprises monomer D, said monomer D being a cell-binding amino acid sequence, preferably wherein monomer D comprises a sequence selected from the list consisting of: RGD (SEQ ID NO: 9), LDT (SEQ ID NO: 27), SEQ ID NO: 10, SEQ ID NO: 17, SEQ ID NO: 18, SEQ ID NO: 19, SEQ ID NO: 28 or SEQ ID NO: 29, more preferably wherein monomer D comprises SEQ ID NO: 6.

3. The composition according to any of claims 1 to 2, wherein the biopolymer has structure (I):

$$[(B_b\text{-}C_c)\text{-}Z_z]_n\text{-}D_d$$

   wherein B, C and D are defined in claims 1 to 2,
   Z is selected from monomers X and/or Y defined in claim 1,
   b has values of between 5 and 15,
   c has values of between 50 and 70;
   z has values of between 1 and 5
   n has values of between 1 and 5,
   d has values of between 0 and 3, and

   it is **characterized in that** it comprises a first biopolymer of structure (I), wherein Z is SEQ ID NO: 4, and a second biopolymer of structure (I), wherein Z is SEQ ID NO: 5, preferably wherein the first biopolymer is found in the composition at a concentration of between 40-60 % by weight and the second biopolymer is found in the composition at a concentration of between 60-40 % by weight.

4. The composition according to any of claims 1 to 2, wherein the biopolymer has structure (II):

$$Z1_z\text{-}[(B_b\text{-}C_c)\text{-}Z2_z]_n\text{-}D_d,$$

   wherein B, C and D are defined in claims 1 to 2,
   Z1 is an amino acid sequence comprising SEQ ID NO: 4 or SEQ ID NO: 5 and
   Z2 is an amino acid sequence comprising SEQ ID NO: 5 or SEQ ID NO: 4, respectively; preferably Z1 is amino acid sequence SEQ ID NO: 4 and Z2 is amino acid sequence SEQ ID NO: 5;
   b, c, z, n and d are defined in claim 3.

5. The composition according to any of claims 3 to 4, wherein b has a value of 10, c has a value of 60, z has a value of 1, n has a value of 2 and d has a value of 0 or 1.

6. The composition according to claim 2, wherein the biopolymer is selected from SEQ ID NO: 16 or from the combination of biopolymers of SEQ ID NO: 14 and SEQ ID NO: 15.

7. The composition according to any of claims 1 to 6, further comprising cells, bioactive molecules, active ingredients, or combinations thereof.

8. Use of the composition according to any of claims 1 to 7 as a bio-ink, preferably for the 2D or 3D printing of biomaterials.

9. Use of the composition according to any of claims 1 to 7, for the preparation of a support for obtaining organs and/or tissues, or for the encapsulation of active ingredients or cells.

10. A bio-ink comprising the composition according to any of claims 1 to 7.

11. A 3D or 2D biomaterial comprising the composition according to any of claims 1 to 7.

12. The composition according to any of claims 1 to 7 for use as a drug, preferably for tissue regeneration.

**Patentansprüche**

1. Zusammensetzung, umfassend ein Biopolymer, umfassend die Monomere B, C, X und Y, oder mindestens zwei Biopolymere, umfassend jeweils die Monomere B, C und X und die Monomere B, C und Y, wobei

B Wiederholungen des Pentapeptids VPGXG (SEQ ID NO: 7) umfasst, vorzugsweise B eine Aminosäuresequenz ist, die SEQ ID NO: 2 umfasst,
C eine Aminosäuresequenz ist, die SEQ ID NO: 3 umfasst,
X eine Aminosäuresequenz ist, die SEQ ID NO: 4 umfasst, und
Y 1 bis 15 Wiederholungen von SEQ ID NO: 8 umfasst, vorzugsweise Y eine Aminosäuresequenz ist, die SEQ ID NO: 5 umfasst.

2. Zusammensetzung gemäß Anspruch 1, wobei das Biopolymer ferner Monomer D umfasst, wobei das Monomer D eine zellbindende Aminosäuresequenz ist, wobei Monomer D vorzugsweise eine Sequenz umfasst, die aus der Liste ausgewählt ist, die aus Folgendem besteht: RGD (SEQ ID NO: 9), LDT (SEQ ID NO: 27), SEQ ID NO: 10, SEQ ID NO: 17, SEQ ID NO: 18, SEQ ID NO: 19, SEQ ID NO: 28 oder SEQ ID NO: 29, wobei Monomer D noch bevorzugter SEQ ID NO: 6 umfasst.

3. Zusammensetzung gemäß einem der Ansprüche 1 bis 2, wobei das Biopolymer die Struktur (I) aufweist:

$$[(B_b C_c)\text{-}Z_z]_n\text{-}D_d$$

wobei B, C und D in den Ansprüchen 1 bis 2 definiert sind,
Z aus den in Anspruch 1 definierten Monomeren X und/oder Y ausgewählt ist,
b Werte zwischen 5 und 15 aufweist,
c Werte zwischen 50 und 70 aufweist;
z Werte zwischen 1 und 5 aufweist
n Werte zwischen 1 und 5 aufweist,
d Werte zwischen 0 und 3 aufweist und

**dadurch gekennzeichnet ist, dass** sie ein erstes Biopolymer der Struktur (I), wobei Z SEQ ID NO: 4 ist, und ein zweites Biopolymer der Struktur (I) umfasst, wobei Z SEQ ID NO: 5 ist, vorzugsweise wobei das erste Biopolymer in der Zusammensetzung in einer Konzentration zwischen 40-60 Gew.-% aufgefunden wird und das zweite Biopolymer in der Zusammensetzung in einer Konzentration zwischen 60-40 Gew.-% aufgefunden wird.

4. Zusammensetzung gemäß einem der Ansprüche 1 bis 2, wobei das Biopolymer die Struktur (II) aufweist:

$$Z1_z\text{-}[(B_b\text{-}C_c)\text{-}Z2_z]_n\text{-}D_d,$$

wobei B, C und D in den Ansprüchen 1 bis 2 definiert sind,

Z1 eine Aminosäuresequenz ist, die SEQ ID NO: 4 oder SEQ ID NO: 5 umfasst, und

Z2 eine Aminosäuresequenz ist, die jeweils SEQ ID NO: 5 oder SEQ ID NO: 4 umfasst; vorzugsweise Z1 die Aminosäuresequenz SEQ ID NO: 4 ist und Z2 die Aminosäuresequenz SEQ ID NO: 5 ist;

b, c, z, n und d in Anspruch 3 definiert sind.

5. Zusammensetzung gemäß einem der Ansprüche 3 bis 4, wobei b einen Wert von 10 aufweist, c einen Wert von 60 aufweist, z einen Wert von 1 aufweist, n einen Wert von 2 aufweist und d einen Wert von 0 oder 1 aufweist.

6. Zusammensetzung gemäß Anspruch 2, wobei das Biopolymer aus SEQ ID NO: 16 oder aus der Kombination der Biopolymere von SEQ ID NO: 14 und SEQ ID NO: 15 ausgewählt ist.

7. Zusammensetzung gemäß einem der Ansprüche 1 bis 6, ferner umfassend Zellen, bioaktive Moleküle, Wirkstoffe oder Kombinationen davon.

8. Verwendung der Zusammensetzung gemäß einem der Ansprüche 1 bis 7 als eine Biotinte, vorzugsweise für den 2D- oder 3D-Druck von Biomaterialien.

9. Verwendung der Zusammensetzung gemäß einem der Ansprüche 1 bis 7 zur Herstellung eines Trägers, um Organe und/oder Gewebe zu erhalten, oder zur Verkapselung von Wirkstoffen oder Zellen.

10. Biotinte, umfassend die Zusammensetzung gemäß einem der Ansprüche 1 bis 7.

11. 3D- oder 2D-Biomaterial, umfassend die Zusammensetzung gemäß einem der Ansprüche 1 bis 7.

12. Zusammensetzung gemäß einem der Ansprüche 1 bis 7 zur Verwendung als Arzneimittel, vorzugsweise zur Geweberegeneration.

## Revendications

1. Composition comprenant un biopolymère comprenant des monomères B, C, X et Y, ou au moins deux biopolymères comprenant des monomères B, C et X et des monomères B, C et Y, respectivement, dans laquelle

B comprend des répétitions du pentapeptide VPGXG (SEQ ID NO : 7), de préférence B est une séquence d'acides aminés comprenant SEQ ID NO : 2,

C est une séquence d'acides aminés comprenant SEQ ID NO : 3,

X est une séquence d'acides aminés comprenant SEQ ID NO : 4 et

Y comprend 1 à 15 répétitions de SEQ ID NO : 8, de préférence Y est une séquence d'acides aminés comprenant SEQ ID NO : 5.

2. Composition selon la revendication 1, dans laquelle le biopolymère comprend en outre le monomère D, ledit monomère D étant une séquence d'acides aminés de liaison cellulaire, de préférence dans laquelle le monomère D comprend une séquence choisie dans la liste constituée de : RGD (SEQ ID NO : 9), LDT (SEQ ID NO : 27), SEQ ID NO : 10, SEQ ID NO : 17, SEQ ID NO : 18, SEQ ID NO : 19, SEQ ID NO : 28 ou SEQ ID NO : 29, de préférence encore dans laquelle le monomère D comprend SEQ ID NO : 6.

3. Composition selon l'une quelconque des revendications 1 à 2, dans laquelle le biopolymère a la structure (I) :

$$[(B_b\text{-}C_c)\text{-}Z_z]_n\text{-}D_d$$

dans laquelle B, C et D sont tels que définis dans les revendications 1 à 2,

Z est choisi parmi les monomères X et/ou Y tels que définis dans la revendication 1,

b vaut entre 5 et 15,

c vaut entre 50 et 70 ;

z vaut entre 1 et 5

n vaut entre 1 et 5,

d vaut entre 0 et 3 et

elle est **caractérisée en ce qu'**elle comprend un premier biopolymère de structure (I), dans laquelle Z est SEQ ID NO : 4, et un second biopolymère de structure (I), dans laquelle Z est SEQ ID NO : 5, de préférence dans laquelle le premier biopolymère se trouve dans la composition en une concentration comprise entre 40 et 60 % en poids et le second biopolymère se trouve dans la composition en une concentration comprise entre 60 et 40 % en poids.

4. Composition selon l'une quelconque des revendications 1 à 2, dans laquelle le biopolymère a la structure (II) :

$$Z1_z\text{-}[(B_b\text{-}C_c)\text{-}Z2_z]_n\text{-}D_d,$$

dans laquelle B, C et D sont tels que définis dans les revendications 1 à 2,
Z1 est une séquence d'acides aminés comprenant SEQ ID NO : 4 ou SEQ ID NO : 5 et
Z2 est une séquence d'acides aminés comprenant SEQ ID NO : 5 ou SEQ ID NO : 4, respectivement ; de préférence, Z1 est la séquence d'acides aminés SEQ ID NO : 4 et Z2 est la séquence d'acides aminés SEQ ID NO : 5 ;
b, c, z, n et d sont tels que définis dans la revendication 3.

5. Composition selon l'une quelconque des revendications 3 à 4, dans laquelle b vaut 10, c vaut 60, z vaut 1, n vaut 2 et d vaut 0 ou 1.

6. Composition selon la revendication 2, dans laquelle le biopolymère est choisi parmi SEQ ID NO : 16 ou parmi la combinaison de biopolymères de SEQ ID NO : 14 et de SEQ ID NO : 15.

7. Composition selon l'une quelconque des revendications 1 à 6, comprenant en outre des cellules, des molécules bioactives, des principes actifs ou des combinaisons de ceux-ci.

8. Utilisation de la composition selon l'une quelconque des revendications 1 à 7 comme encre biologique, de préférence pour l'impression 2D ou 3D de biomatériaux.

9. Utilisation de la composition selon l'une quelconque des revendications 1 à 7, pour la préparation d'un support pour l'obtention d'organes et/ou de tissus ou pour l'encapsulation de principes actifs ou de cellules.

10. Encre biologique comprenant la composition selon l'une quelconque des revendications 1 à 7.

11. Biomatériau 3D ou 2D comprenant la composition selon l'une quelconque des revendications 1 à 7.

12. Composition selon l'une quelconque des revendications 1 à 7 destinée à être utilisée comme médicament, de préférence pour la régénération tissulaire.

**Fig. 1**

**Fig. 2**

**Fig. 3**

**Fig. 4**

**Fig. 5**

**Fig. 6**

**Fig. 7**

**Fig. 8**

**Fig. 9**

**Fig. 10**

**Fig. 11**

**Fig. 12**

**Fig. 13**

**Fig. 14**

**Fig. 15**

**Fig. 16**

**Fig. 17**

**Fig. 18**

Fig. 19

Fig. 20

**Fig. 21**

**Fig. 22**

**Fig. 23**

**Fig. 24**

**A**

**B**

300 mg/mL

250 mg/mL

200 mg/mL

180 mg/mL

150 mg/mL

120 mg/mL

**Fig. 25**

**FIG. 26**

BP 1 (60%) + BP 5
(40%)

BP 1 (40%) + BP 4
(60%)

BP 4 (60%) + BP 5
(40%)

BP 4 (80%) + pre-cured
BP 5 (20%)

BP 4 (60%) + pre-cured
BP 5 (40%)

FIG. 26 (cont.)

**FIG. 27**

**FIG. 28**

FIG. 29

| RECENTLY PRINTED | 1 DAY | 2 DAYS | 3 DAYS |

FIG. 30

**PRINTING**  **RECENTLY PRINTED**  **2 DAYS**

**FIG. 31**

**RECENTLY PRINTED**  **1 DAY**  **3 DAYS**

**7 DAYS**  **22 DAYS**  **40 DAYS**

**FIG. 32**

| RECENTLY PRINTED | 1 DAY | 3 DAYS | 40 DAYS |
|---|---|---|---|

**FIG. 33**

**FIG. 34**

FIG. 35

FIG. 36

**FIG. 37**

**FIG. 38**

**FIG. 39**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010092224 A

**Non-patent literature cited in the description**

- **OZBOLAT, I.T. et al.** *Drug Discovery Today,* 2016, vol. 21 (8), 1257-1271
- **VANDERBURGH, J. et al.** *Ann Biomed Eng,* 2017, vol. 45 (1), 164-179
- **JUNGST, T. et al.** *Chemical Reviews,* 2016, vol. 116 (3), 1496-1539
- **CHIMENE, D. et al.** *Ann Biomed Eng.,* 2016, vol. 44 (6), 2090-2102